# EUROPEAN PATENT SPECIFICATION

(11) **EP 2 612 662 B1**
(45) Date of publication and mention of the grant of the patent: **04.03.2020**
(21) Application number: 12178541.4
(22) Date of filing: 30.07.2012
(51) Int. Cl.: A61K 31/12, A61K 31/167, A61K 31/192, A61K 31/405, A61K 31/616, A61K 45/06, A61P 7/12, A61P 13/10, A61K 31/216, A61K 31/4025, A61K 31/4725, A61K 31/46, A61K 9/00

(54) **Delayed-release formulation for reducing the frequency of urination and method of use thereof**
Formulierung mit verzögerter Freisetzung zur Verringerung der Blasenentleerungshäufigkeit und Verwendungsverfahren dafür
Formulation à libération retardée pour réduire la fréquence de la miction et son procédé d'utilisation

(30) Priority: 04.06.2012 US 201213487343; 19.03.2012 US 201213423949; 04.01.2012 US 201213343332; 04.01.2012 US 201213343349
(43) Date of publication of application: 10.07.2013
(73) Proprietor: Wellesley Pharmaceuticals, LLC, Newtown, PA 18940 (US)
(72) Inventor: Dill, David A., Newtown, PA Pennsylvania 18940 (US)
(74) Representative: TL Brand & Co

(56) References cited:
- EP-A1- 1 053 752
- EP-A1- 1 627 876
- WO-A1-01/19350
- WO-A1-03/070233
- WO-A1-2010/138441
- WO-A2-2011/107755
- US-A1- 2004 054 008
- US-A1- 2004 198 822
- US-A1- 2008 085 314
- PINNA C ET AL: "Altered neurogenic and mechanical responses to acetylcholine, ATP and substance P in detrusor from rat with outlet obstruction", LIFE SCIENCES, PERGAMON PRESS, OXFORD, GB, vol. 79, no. 13, 22 August 2006 (2006-08-22), pages 1301-1306, XP028051173, ISSN: 0024-3205, DOI: 10.1016/J.LFS.2006.03.035 [retrieved on 2006-08-22]
- ADDLA S K ET AL: "Diclofenac for Treatment of Nocturia Caused by Nocturnal Polyuria: A Prospective, Randomised, Double-Blind, Placebo-Controlled Crossover Study", EUROPEAN UROLOGY, ELSEVIER BV, NL, vol. 49, no. 4, 1 April 2006 (2006-04-01), pages 720-726, XP027881854, ISSN: 0302-2838 [retrieved on 2006-04-01]
- WEIN A J ET AL: "Overactive Bladder: A Better Understanding of Pathophysiology, Diagnosis and Management", JOURNAL OF UROLOGY, LIPPINCOTT WILLIAMS & WILKINS, BALTIMORE, MD, US, vol. 175, no. 3, 1 March 2006 (2006-03-01) , pages S5-S10, XP024989968, ISSN: 0022-5347, DOI: 10.1016/S0022-5347(05)00313-7 [retrieved on 2006-03-01]
- WEISS ET AL: "NOCTURIA", JOURNAL OF UROLOGY, LIPPINCOTT WILLIAMS & WILKINS, BALTIMORE, MD, US, vol. 163, no. 1, 1 January 2000 (2000-01-01), pages 5-12, XP005556162, ISSN: 0022-5347, DOI: 10.1016/S0022-5347(05)67961-X
- MATTHIESEN ET AL: "Nocturnal Polyuria and Natriuresis in Male Patients with Nocturia and Lower Urinary Tract Symptoms", JOURNAL OF UROLOGY, LIPPINCOTT WILLIAMS & WILKINS, BALTIMORE, MD, US, vol. 156, no. 4, 1 October 1996 (1996-10-01), pages 1292-1299, XP005573755, ISSN: 0022-5347, DOI: 10.1016/S0022-5347(01)65572-1

## Description

This application claims priority from the U.S. Patent Application Serial No. 13/487,343, filed on June 4, 2012, U.S. Patent Application Serial No 13/423,949, filed on March 19, 2012, U.S. Patent Application Serial Nos. 13/343,332 and 13/343,349, filed on January 4, 2012.

### FIELD

The present application generally discloses methods and compositions for inhibiting the contraction of muscles and, in particular, methods and compositions for inhibiting the contraction of smooth muscles of the urinary bladder.

### BACKGROUND

The detrusor muscle is a layer of the urinary bladder wall made of smooth muscle fibers arranged in spiral, longitudinal, and circular bundles. When the bladder is stretched, this signals the parasympathetic nervous system to contract the detrusor muscle. This encourages the bladder to expel urine through the urethra.

For the urine to exit the bladder, both the autonomically controlled internal sphincter and the voluntarily controlled external sphincter must be opened. Problems with these muscles can lead to incontinence. If the amount of urine reaches 100% of the urinary bladder's absolute capacity, the voluntary sphincter becomes involuntary and the urine will be ejected instantly.

The human adult urinary bladder usually holds about 300-350 ml of urine (the working volume), but a full adult bladder may hold up to about 1000 ml (the absolute volume), varying among individuals. As urine accumulates, the ridges produced by folding of the wall of the bladder (rugae) flatten and the wall of the bladder thins as it stretches, allowing the bladder to store larger amounts of urine without a significant rise in internal pressure.

In most individuals, the desire to urinate usually starts when the volume of urine in the bladder reaches around 200 ml. At this stage it is easy for the subject, if desired, to resist the urge to urinate. As the bladder continues to fill, the desire to urinate becomes stronger and harder to ignore. Eventually, the bladder will fill to the point where the urge to urinate becomes overwhelming, and the subject will no longer be able to ignore it. In some individuals, this desire to urinate starts when the bladder is less than 100% full in relation to its working volume. Such increased desire to urinate may interfere with normal activities, including the ability to sleep for sufficient uninterrupted periods of rest. In some cases, this increased desire to urinate may be associated with medical conditions such as benign prostate hyperplasia or prostate cancer in men, or pregnancy in women. However, increased desire to urinate also occurs in individuals, both male and female, who are not affected by another medical condition.

Accordingly, there exists a need for compositions and methods for the treatment of male and female subjects who suffer from a desire to urinate when the bladder is less than 100% full of urine in relation to its working volume. Said compositions and methods are needed for the inhibition of muscle contraction in order to allow in said subjects the desire to urinate to start when the volume of urine in the bladder exceeds around 100% of its working volume.

Addla et al. (European Urology, 2006, 49, 720-726) describes the use of non-steroidal anti-inflammatory drugs in the treatment of nocturnal polyuria.

### SUMMARY

One aspect of the present application relates to a pharmaceutical composition for use in therapeutically reducing the frequency of urination, comprising a first analgesic agent, wherein said first analgesic agent is acetaminophen, wherein said pharmaceutical composition is formulated in a delayed-release formulation having an enteric coating, and wherein said first analgesic agent is administered orally at a daily dose of 5 mg to 2000 mg.

Also discussed herein is a method which comprises administering to a subject in need thereof an effective amount of a pharmaceutical composition comprising a plurality of active ingredients, wherein the plurality of active ingredients comprises (1) one or more analgesic agents and/or (2) one or more antimuscarinic agents and wherein the one or more analgesic agents are administered orally at a combined daily dose of 5 mg to 2000 mg. In some arrangements, the one or more analgesic agents are selected from the group consisting of aspirin, ibuprofen, naproxen sodium and acetaminophen. Examples of the antimuscarinic agents include, but are not limited to, oxybutynin, solifenacin, darifenacin, fesoterodine, tolterodine, trospium and atropine.

Also discussed herein is a pharmaceutical composition, comprising: one or more analgesic agents selected from the group consisting of aspirin, ibuprofen, naproxen, and acetaminophen; one or more antidiuretics; and a pharmaceutically acceptable carrier, wherein at least one of said one or more analgesic agents is formulated for delayed-release.

### BRIEF DESCRIPTION OF DRAWINGS

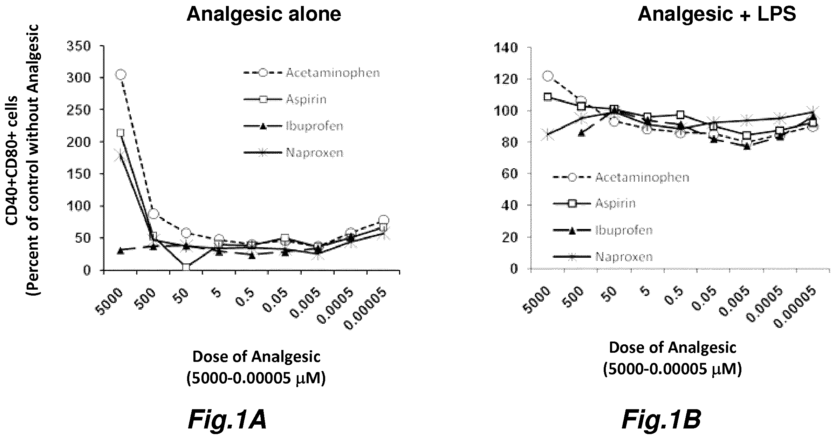

Figure 1A and 1B are diagrams showing that analgesics regulate expression of co-stimulatory molecules by Raw 264 macrophage cells in the absence (Figure 1A) or presence (Figure 1B) of LPS. Cells were cultures for 24 hrs in the presence of analgesic alone or together with *Salmonella typhyiurium* LPS (0.05 µg/ml). Results are mean relative % of CD40+CD80+ cells.

### DETAILED DESCRIPTION

The following detailed description is presented to enable any person skilled in the art to make and use the invention. For purposes of explanation, specific nomenclature is set forth to provide a thorough understanding of the present invention. However, it will be apparent to one skilled in the art that these specific details are not required to practice the invention. Descriptions of specific applications are provided only as representative examples. The present invention is not intended to be limited to the embodiments shown, but is to be accorded the broadest possible scope consistent with the principles and features disclosed herein.

As used herein, the term "effective amount" means an amount necessary to achieve a selected result.

As used herein, the term "analgesic" refers to agents, compounds or drugs used to relieve pain and inclusive of anti-inflammatory compounds. Exemplary analgesic and/or anti-inflammatory agents, compounds or drugs include, but are not limited to, the following substances: non-steroidal anti-inflammatory drugs (NSAIDs), salicylates, aspirin, salicylic acid, methyl salicylate, diflunisal, salsalate, olsalazine, sulfasalazine, para-aminophenol derivatives, acetanilide, acetaminophen, phenacetin, fenamates, mefenamic acid, meclofenamate, sodium meclofenamate, heteroaryl acetic acid derivatives, tolmetin, ketorolac, diclofenac, propionic acid derivatives, ibuprofen, naproxen sodium, naproxen, fenoprofen, ketoprofen, flurbiprofen, oxaprozin; enolic acids, oxicam derivatives, piroxicam, meloxicam, tenoxicam, ampiroxicam, droxicam, pivoxicam, pyrazolon derivatives, phenylbutazone, oxyphenbutazone, antipyrine, aminopyrine, dipyrone, coxibs, celecoxib, rofecoxib, nabumetone, apazone, indomethacin, sulindac, etodolac, isobutylphenyl propionic acid, lumiracoxib, etoricoxib, parecoxib, valdecoxib, tiracoxib, etodolac, darbufelone, dexketoprofen, aceclofenac, licofelone, bromfenac, pranoprofen, loxoprofen, piroxicam, nimesulide, cizolirine, 3-formylamino-7-methylsulfonylamino-6-phenoxy-4H-1-benzopyran-4-one, meloxicam, lornoxicam, d-indobufen, mofezolac, amtolmetin, pranoprofen, tolfenamic acid, flurbiprofen, suprofen, oxaprozin, zaltoprofen, alminoprofen, tiaprofenic acid, pharmacological salts thereof, hydrates thereof, and solvates thereof.

As used herein, the terms "coxib" and "COX inhibitor" refer to a composition of compounds that is capable of inhibiting the activity or expression of COX2 enzymes or is capable of inhibiting or reducing the severity, including pain and swelling, of a severe inflammatory response.

The urinary bladder has two important functions: storage of urine and emptying. Storage of urine occurs at low pressure, which implies that the detrusor muscle relaxes during the filling phase. Emptying of the bladder requires a coordinated contraction of the detrusor muscle and relaxation of the sphincter muscles of the urethra. Disturbances of the storage function may result in lower urinary tract symptoms, such as urgency, frequency, and urge incontinence, the components of the overactive bladder syndrome. The overactive bladder syndrome, which may be due to involuntary contractions of the smooth muscle of the bladder (detrusor) during the storage phase, is a common and underreported problem, the prevalence of which has only recently been assessed.

Discussed herein is a method for reducing the frequency of urination by administering to a person in need thereof a pharmaceutical composition formulated in a delayed-release formulation. The pharmaceutical composition comprises one or more analgesic agents and, optionally, one or more antimuscarinic agents.

As used herein, the term "delayed-release" refers to a medication that does not immediately disintegrate and release the active ingredient(s) into the body. In some embodiments, the term "delayed-release" is used with reference to a drug formulation having a release profile in which there is a predetermined delay in the release of the drug following administration. The delayed-release formulation includes an enteric coating, which is a barrier applied to oral medication that prevents release of medication before it reaches the small intestine. Delayed-release formulations, such as enteric coatings, prevent drugs having an irritant effect on the stomach, such as aspirin, from dissolving in the stomach. Such coatings are also used to protect acid-unstable drugs from the stomach's acidic exposure, delivering them instead to a basic pH environment (intestine's pH 5.5 and above) where they do not degrade, and give their desired action.

The term "pulsatile release" is a type of delayed-release, which is used herein with reference to a drug formulation that provides rapid and transient release of the drug within a short time period immediately after a predetermined lag period, thereby producing a "pulsed" plasma profile of the drug after drug administration. Formulations may be designed to provide a single pulsatile release or multiple pulsatile releases at predetermined time intervals following administration.

Most enteric coatings work by presenting a surface that is stable at the highly acidic pH found in the stomach, but breaks down rapidly at a less acidic (relatively more basic) pH. Therefore, an enteric coated pill will not dissolve in the acidic juices of the stomach (pH ∼3), but they will in the alkaline (pH 7-9) environment present in the small intestine. Examples of enteric coating materials include, but are not limited to, methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In some embodiments, the pharmaceutical composition is orally administered from a variety of drug formulations designed to provide delayed-release. Delayed oral dosage forms include, for example, tablets, capsules, caplets, and may also comprise a plurality of granules, beads, powders or pellets that may or may not be encapsulated. Tablets and capsules represent the most convenient oral dosage forms, in which case solid pharmaceutical carriers are employed.

In a delayed-release formulation, one or more barrier coatings may be applied to pellets, tablets, or capsules to facilitate slow dissolution and concomitant release of drugs into the intestine. Typically, the barrier coating contains one or more polymers encasing, surrounding, or forming a layer, or membrane around the therapeutic composition or active core.

In some embodiments, the active agents are delivered in a formulation to provide delayed-release at a pre-determined time following administration. The delay may be up to about 10 minutes, about 20 minutes, about 30 minutes, about 1 hour, about 2 hours, about 3 hours, about 4 hours, about 5 hours, about 6 hours, or longer.

A delayed-release composition may comprise 100% of the total dosage of a given active agent administered in a single unit dose. Alternatively, a delayed-release composition may be included as a component in a combined release profile formulation may provide about 30-95% of the total dosage of the active agent(s) to be delivered by the pharmaceutical formulation. For example, the immediate-release component may provide about 5-70%, or about 50% of the total dosage of the active agent(s) to be delivered by the pharmaceutical formulation. In alternate embodiments, the delayed-release component provides about 30, 35, 40, 45, 50, 55, 60, 65, 70, 75, 80, 85, 90 or 95% of the total dosage of the active agent(s) to be delivered by the formulation.

A delayed-release formulation typically comprises a barrier coating that delays the release of the active ingredient(s). The barrier coating may consist of a variety of different materials, depending on the objective. In addition, a formulation may comprise a plurality of barrier coatings to facilitate release in a temporal manner. The coating may be a sugar coating, a film coating (*e.g*., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or a coating based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose. Furthermore, the formulation may additionally include a time delay material such as, for example, glyceryl monostearate or glyceryl distearate.

In some embodiments, the delayed-release formulation includes an enteric coating comprised one or more polymers facilitating release of active agents in proximal or distal regions of the gastrointestinal tract. As used herein, the term "enteric polymer coating" a coating comprising of one or more polymers having a pH dependent or pH-independent release profile. Typically the coating resists dissolution in the acidic medium of the stomach, but dissolves or erodes in more distal regions of the gastrointestinal tract, such as the small intestine or colon. An enteric polymer coating typically resists releases of the active agents until some time after a gastric emptying lag period of about 3-4 hours after administration.

pH dependent enteric coatings comprising one or more pH-dependent or pH-sensitive polymers that maintain their structural integrity at low pH, as in the stomach, but dissolve in higher pH environments in more distal regions of the gastrointestinal tract, such as the small intestine, where the drug contents are released. For purposes of the present invention, "pH dependent" is defined as having characteristics (e.g., dissolution) which vary according to environmental pH. Exemplary pH-dependent polymers include, but are not limited to, methacarylic acid copolymers, methacrylic acid-methyl methacrylate copolymers (e.g., EUDRAGIT® L100 (Type A), EUDRAGIT® S100 (Type B), Rohm GmbH, Germany; methacrylic acid-ethyl acrylate copolymers (*e.g*., EUDRAGIT® L100-55 (Type C) and EUDRAGIT® L30D-55 copolymer dispersion, Rohm GmbH, Germany); copolymers of methacrylic acid-methyl methacrylate and methyl methacrylate (EUDRAGIT® FS); terpolymers of methacrylic acid, methacrylate, and ethyl acrylate; cellulose acetate phthalates (CAP); hydroxypropyl methylcellulose phthalate (HPMCP) (*e.g.,* HP-55, HP-50, HP-55S, Shinetsu Chemical, Japan); polyvinyl acetate phthalates (PVAP) (*e.g*., COATERIC®, OPADRY® enteric white OY-P-7171); cellulose acetate succinates (CAS); hydroxypropyl methylcellulose acetate succinate (HPMCAS), *e.g.,* HPMCAS LF Grade, MF Grade, HF Grade, including AQOAT® LF and AQOAT® MF (Shin-Etsu Chemical, Japan); Shinetsu Chemical, Japan); shellac (e.g., Marcoat™ 125 & Marcoat™ 125N); carboxymethyl ethylcellulose (CMEC, Freund Corporation, Japan), cellulose acetate phthalates (CAP)(*e.g*., AQUATERIC®); cellulose acetate trimellitates (CAT); and mixtures of two or more thereof at weight ratios between about 2:1 to about 5:1, such as, for instance, a mixture of EUDRAGIT® L 100-55 and EUDRAGIT® S 100 at a weight ratio of about 3:1 to about 2:1, or a mixture of EUDRAGIT® L 30 D-55 and EUDRAGIT® FS at a weight ratio of about 3:1 to about 5:1.

pH-dependent polymers typically exhibit a characteristic pH optimum for dissolution. In some embodiments, the pH-dependent polymer exhibits a pH optimum between about 5.0 and 5.5, between about 5.5 and 6.0, between about 6.0 and 6.5, or between about 6.5 and 7.0. In other embodiments, the pH-dependent polymer exhibits a pH optimum of ≥5.0, of ≥5.5, of ≥6.0, of ≥6.5, or of ≥7.0.

In other embodiments, the enteric coating may comprise one or more pH-independent polymers. These polymers provide for release of the drug after a certain time, independent of the pH. For purposes of the present invention, "pH independent" is defined as having characteristics (*e.g*., dissolution) which are substantially unaffected by pH. pH independent polymers are often referred to in the context of "time-controlled" or "time-dependent" release profiles.

A pH independent polymer may be water-insoluble or water soluble. Exemplary water insoluble pH independent polymers include, but are not limited to, neutral methacrylic acid esters with a small portion of trimethylammonioethyl methacrylate chloride (*e.g*., EUDRAGIT® RS and EUDRAGIT® RL; neutral ester dispersions without any functional groups (*e.g*., EUDRAGIT® NE30D and EUDRAGIT®NE30); cellulosic polymers, such as ethylcellulose, hydroxyl ethyl cellulose, cellulose acetate or mixtures and other pH independent coating products. Exemplary water soluble pH independent polymers include OPADRY®amb.

In some embodiments, the pH independent polymers contain one or more polysaccharides that are resistant to erosion in both the stomach and intestine. Such polymers can be only degraded in the colon, which contains a large microflora containing biodegradable enzymes breaking down, for example, the polysaccharide coatings to release the drug contents in a controlled, time-dependent manner.

In certain embodiment, the coating methodology employs the blending of one or more pH-dependent and one or more pH-independent polymers. The blending of pH-dependent and pH-independent polymers can reduce the release rate of active ingredients once the soluble polymer has reached its optimum pH of solubilization.

In some embodiments, a "time-controlled" or "time-dependent" release profile can be obtained using a water insoluble capsule body containing one or more active agents, wherein the capsule body closed at one end with an insoluble, but permeable and swellable hydrogel plug. Upon contact with gastrointestinal fluid or dissolution medium, the plug swells, pushing itself out of the capsule and releasing the drugs after a pre-determined lag time, which can be controlled by *e.g.,* the position and dimensions of the plug. The capsule body may be further coated with an outer pH-dependent enteric coating keeping the capsule intact until it reaches the small intestine. Suitable plug materials include, for example, polymethacrylates, erodible compressed polymers (*e.g*., HPMC, polyvinyl alcohol), congealed melted polymer (*e.g*., glyceryl mono oleate) and enzymatically controlled erodible polymers (*e.g*., polysaccharides, such as amylose, arabinogalactan, chitosan, chondroitin sulfate, cyclodextrin, dextran, guar gum, pectin and xylan).

In other embodiments, capsules or bilayered tablets may be formulated to contain a drug-containing core, covered by a swelling layer, and an outer insoluble, but semi-permeable polymer coating or membrane. The lag time prior to rupture can be controlled by the permeation and mechanical properties of the polymer coating and the swelling behavior of the swelling layer. Typically, the swelling layer comprises one or more swelling agents, such as swellable hydrophilic polymers that swell and retain water in their structures.

Exemplary water swellable materials include, but are not limited to, polyethylene oxide (having *e.g.,* an average molecular weight between 1,000,000 to 7,000,000, such as POLYOX®), methylcellulose, hydroxypropyl cellulose, hydroxypropyl methylcellulose; polyalkylene oxides having a weight average molecular weight of 100,000 to 6,000,000, including but not limited to poly(methylene oxide), poly(butylene oxide); poly(hydroxy alkyl methacrylate) having a molecular weight of from 25,000 to 5,000,000; poly(vinyl)alcohol, having a low acetal residue, which is cross-linked with glyoxal, formaldehyde or glutaraldehyde and having a degree of polymerization of from 200 to 30,000; mixtures of methyl cellulose, cross-linked agar and carboxymethyl cellulose; hydrogel forming copolymers produced by forming a dispersion of a finely divided copolymer of maleic anhydride with styrene, ethylene, propylene, butylene or isobutylene cross-linked with from 0.001 to 0.5 moles of saturated cross-linking agent per mole of maleic anyhydride in the copolymer; CARBOPOL® acidic carboxy polymers having a molecular weight of 450,000 to 4,000,000; CYANAMER® polyacrylamides; cross-linked water swellable indenemaleicanhydride polymers; GOODRITE® polyacrylic acid having a molecular weight of 80,000 to 200,000; starch graft copolymers; AQUA-KEEPS® acrylate polymer polysaccharides composed of condensed glucose units such as diester cross-linked polyglucan; carbomers having a viscosity of 3,000 to 60,000 mPa as a 0.5%-1% w/v aqueous solution; cellulose ethers such as hydroxypropylcellulose having a viscosity of about 1000-7000 mPa s as a 1% w/w aqueous solution (25° C); hydroxypropyl methylcellulose having a viscosity of about 1000 or higher, preferably 2,500 or higher to a maximum of 25,000 mPa as a 2% w/v aqueous solution; polyvinylpyrrolidone having a viscosity of about 300-700 mPa s as a 10% w/v aqueous solution at 20° C; and combinations thereof

The enteric layer may further comprise anti-tackiness agents, such as talc or glyceryl monostearate and/or plasticizers such as. The enteric layer may further comprise one or more plasticizers including, but not limited to, triethyl citrate, acetyl triethyl citrate, acetyltributyl citrate, polyethylene glycol acetylated monoglycerides, glycerin, triacetin, propylene glycol, phthalate esters (e.g., diethyl phthalate, dibutyl phthalate), titanium dioxide, ferric oxides, castor oil, sorbitol and dibutyl sebacate.

In another embodiment, the delay release formulation employs a water-permeable but insoluble film coating to enclose the active ingredient and an osmotic agent utilizing a enclosing. As water from the gut slowly diffuses through the film into the core, the core swells until the film bursts, thereby releasing the active ingredients. The film coating may be adjusted to permit various rates of water permeation or release time.

Alternatively, the release time of the drugs can be controlled by a disintegration lag time depending on the balance between the tolerability and thickness of a water insoluble polymer membrane (such as ethyl cellulose, EC) containing predefined micropores at the bottom of the body and the amount of a swellable excipient, such as low substituted hydroxypropyl cellulose (L-HPC) and sodium glycolate. After oral administration, GI fluids permeate through the micropores, causing swelling of the swellable excipients, which produces an inner pressure disengaging the capsular components, including a first capsule body containing the swellable materials, a second capsule body containing the drugs, and an outer cap attached to the first capsule body.

In other embodiments, the drugs may be released by an osmotic mechanism. By way of example, a capsule may be formulated with a single osmotic unit or it may incorporate 2, 3, 4, 5, or 6 push-pull units encapsulated within a hard gelatin capsule, whereby each bilayer push pull unit contains an osmotic push layer and a drug layer, both surrounded by a semi-permeable membrane. One or more orifices are drilled through the membrane next to the drug layer. This membrane may be additionally covered with a pH-dependent enteric coating to prevent release until after gastric emptying. The gelatin capsule dissolves immediately after ingestion. As the push pull unit(s) enter the small intestine, the enteric coating breaks down, which then allows fluid to flow through the semi-permeable membrane, swelling the osmotic push compartment to force to force drugs out through the orifice(s) at a rate precisely controlled by the rate of water transport through the semi-permeable membrane. Release of drugs can occur over a constant rate for up to 24 hours or more.

The osmotic push layer comprises one or more osmotic agents creating the driving force for transport of water through the semi-permeable membrane into the core of the delivery vehicle. One class of osmotic agents includes water-swellable hydrophilic polymers, also referred to as "osmopolymers" and "hydrogels," including, but not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl, cellulose (CEC), sodium alginate, polycarbophil, gelatin, xanthan gum, and sodium starch glycolate.

Another class of osmotic agents includes osmogens, which are capable of imbibing water to effect an osmotic pressure gradient across the semi-permeable membrane. Exemplary osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol; organic acids, such as ascorbic acid, benzoic acid, fumaric acid, citric acid, maleic acid, sebacic acid, sorbic acid, adipic acid, edetic acid, glutamic acid, p-toluenesulfonic acid, succinic acid, and tartaric acid; urea; and mixtures thereof.

Materials useful in forming the semipermeable membrane include various grades of acrylics, vinyls, ethers, polyamides, polyesters, and cellulosic derivatives that are water-permeable and water-insoluble at physiologically relevant pHs, or are susceptible to being rendered water-insoluble by chemical alteration, such as crosslinking.

In another embodiment, the delay release formulation employs a water-impermeable tablet coating whereby water enters through a controlled aperture in the coating until the core bursts. When the tablet bursts, the drug contents are released immediately or over a longer period of time. These and other techniques may be modified to allow for a pre-determined lag period before release of drugs is initiated.

Various coating techniques may be applied to granules, beads, powders or pellets, tablets, capsules or combinations thereof containing active agents to produce different and distinct release profiles. In some embodiments, the pharmaceutical composition is in a tablet or capsule form containing a single coating layer. In other embodiments, the pharmaceutical composition is in a tablet or capsule form containing multiple coating layers.

In some embodiments, the pharmaceutical composition comprises a plurality of active ingredients selected from the group consisting of analgesics, antimuscarinic agents, antidiuretics and spasmolytics. Examples of spasmolytics include, but are not limited to, carisoprodol, benzodiazepines, baclofen, cyclobenzaprine, metaxalone, methocarbamol, clonidine, clonidine analog, and dantrolene. The pharmaceutical composition comprises one or more analgesics. In other embodiments, the pharmaceutical composition comprises (1) one or more analgesics, and (2) one or more other active ingredients selected from the group consisting of antimuscarinic agents, antidiuretics and spasmolytics. In another embodiment, the pharmaceutical composition comprises (1) one or two analgesics and (2) one or two antimuscarinic agents. In another embodiment, the pharmaceutical composition comprises (1) one or two analgesics and (2) one or two antidiuretics. In another embodiment, the pharmaceutical composition comprises (1) one or two analgesics and (2) one or two spasmolytics. In yet another embodiment, the pharmaceutical composition comprises (1) one or two analgesics, (2) one or two antimuscarinic agents, and (3) one or two antidiuretics.

In one embodiment, the plurality of active ingredients are formulated for delayed-release. In other embodiment, the plurality of active ingredients are formulated for both immediate-release and delayed-release (*e.g*., a first portion of each active ingredient is formulated for immediate-release and a second portion of each active ingredient is formulated for delayed-release). In yet other embodiment, some of the plurality of active ingredients are formulated for immediate-release and some of the plurality of active ingredients are formulated for delayed-release (*e.g*., active ingredients A, B, C are formulated for immediate-release and active ingredients C and D are formulated for delayed-release).

In certain embodiments, the pharmaceutical composition comprises an immediate-release component and a delayed-release component. The immediate-release component may comprise one or more active ingredients selected from the group consisting of analgesics, antimuscarinic agents, antidiuretics and spasmolytics. The delayed-release component may comprise one or more active ingredients selected from the group consisting of analgesics, antimuscarinic agents, antidiuretics and spasmolytics. In some embodiments, the immediate-release component and the delayed-release component have exactly the same active ingredients. In other embodiments, the immediate-release component and the delayed-release component have different active ingredients. In yet other embodiments, the immediate-release component and the delayed-release component have one or more common active ingredients.

In one embodiment, the pharmaceutical composition comprises two active ingredients (*e.g*., two analgesic agents, or a mixture of one analgesic agent and one antimuscarinic agent or antidiuretic or spasmolytic), formulated for immediate-release at about the same time. In another embodiment, the pharmaceutical composition comprises two active ingredients, formulated for delayed-release at about the same time. In another embodiment, the pharmaceutical composition comprises two active ingredients formulated as two delayed-release components, each providing a different delayed-release profile. For example, a first delayed-release component releases a first active ingredient at a first time point and a second delayed-release component releases a second active ingredient at a second time point. In another embodiment, the pharmaceutical composition comprises two active ingredients, one is formulated for immediate-release and the other is formulated for delayed-release.

In other embodiments, the pharmaceutical composition comprises two active ingredients (*e.g*., two analgesic agents, or a mixture of one analgesic agent and one antimuscarinic agent or antiuretic or spasmolytic) formulated for immediate-release, and (2) two active ingredients (*e.g*., two analgesic agents, or a mixture of one analgesic agent and one antimuscarinic agent or antiuretic or spasmolytic) formulated for delayed-release. In other embodiments, the pharmaceutical composition comprises three active ingredients formulated for immediate-release, and (2) three active ingredients formulated for delayed-release. In other embodiments, the pharmaceutical composition comprises four active ingredients formulated for immediate-release, and (2) four active ingredients formulated for delayed-release. In these embodiments, the active ingredient(s) in the immediate-release component can be the same as, or different from, the active ingredient(s) in the delayed-release component.

The term "immediate-release" is used herein with reference to a drug formulation that does not contain a dissolution rate controlling material. There is substantially no delay in the release of the active agents following administration of an immediate-release formulation. An immediate-release coating may include suitable materials immediately dissolving following administration so as to release the drug contents therein. Exemplary immediate-release coating materials include gelatin, polyvinyl alcohol polyethylene glycol (PVA-PEG) copolymers (*e.g*., KOLLICOAT®) and various others materials known to those skilled in the art.

An immediate-release composition may comprise 100% of the total dosage of a given active agent administered in a single unit dose. Alternatively, an immediate-release component may be included as a component in a combined release profile formulation that may provide about 1% to about 50% of the total dosage of the active agent(s) to be delivered by the pharmaceutical formulation. For example, the immediate-release component may provide at least about 5%, or about 10% to about 30%, or about 45% to about 50% of the total dosage of the active agent(s) to be delivered by the formulation. The rest of the active agent(s) may be delivered in a delayed-release formulation. In alternate embodiments, the immediate-release component provides about 10, 15, 20, 25, 30, 35, 40, 45 or 50% of the total dosage of the active agent(s) to be delivered by the formulation. The delayed-release component provides about 90, 85, 80, 75, 70, 65, 60, 55 or 50% of the total dosage of the active agent(s) to be delivered by the formulation.

In some embodiments, the immediate-release or delayed-release formulation comprises an active core comprised of one or more inert particles, each in the form of a bead, pellet, pill, granular particle, microcapsule, microsphere, microgranule, nanocapsule, or nanosphere coated on its surfaces with drugs in the form of *e.g.,* a drug-containing film-forming composition using, for example, fluid bed techniques or other methodologies known to those of skill in the art. The inert particle can be of various sizes, so long as it is large enough to remain poorly dissolved. Alternatively, the active core may be prepared by granulating and milling and/or by extrusion and spheronization of a polymer composition containing the drug substance.

The amount of drug in the core will depend on the dose that is required, and typically varies from about 5 to 90 weight %. Generally, the polymeric coating on the active core will be from about 1 to 50% based on the weight of the coated particle, depending on the lag time and type of release profile required and/or the polymers and coating solvents chosen. Those skilled in the art will be able to select an appropriate amount of drug for coating onto or incorporating into the core to achieve the desired dosage. In one embodiment, the inactive core may be a sugar sphere or a buffer crystal or an encapsulated buffer crystal such as calcium carbonate, sodium bicarbonate, fumaric acid, tartaric acid, etc. which alters the microenvironment of the drug to facilitate its release.

In some embodiments, the delayed-release formulation is formed by coating a water soluble/dispersible drug-containing particle, such as a bead, with a mixture of a water insoluble polymer and an enteric polymer, wherein the water insoluble polymer and the enteric polymer may be present at a weight ratio of from 4:1 to 1:1, and the total weight of the coatings is 10 to 60 weight % based on the total weight of the coated beads. The drug layered beads may optionally include an inner dissolution rate controlling membrane of ethylcellulose. The composition of the outer layer, as well as the individual weights of the inner and outer layers of the polymeric membrane are optimized for achieving desired circadian rhythm release profiles for a given active, which are predicted based on *in vitro*/*in vivo* correlations.

In other embodiments the formulations comprise a mixture of immediate-release drug-containing particles without a dissolution rate controlling polymer membrane and delayed-release beads exhibiting, for example, a lag time of 2-4 hours following oral administration, thus providing a two-pulse release profile. In yet other embodiments the formulations comprise a mixture of two types of delayed-release beads: a first type that exhibits a lag time of 1-3 hours and a second type that exhibits a lag time of 4-6 hours.

In some embodiments, the active core is coated with one or more layers of dissolution rate-controlling polymers to obtain desired release profiles with or without a lag time. An inner layer membrane can largely control the rate of drug release following imbibition of water or body fluids into the core, while the outer layer membrane can provide for a desired lag time (the period of no or little drug release following imbibition of water or body fluids into the core). The inner layer membrane may comprise a water insoluble polymer, or a mixture of water insoluble and water soluble polymers.

The polymers suitable for the outer membrane, which largely controls the lag time of up to 6 hours may comprise an enteric polymer, as described above, and a water insoluble polymer at 10 to 50 weight %. The ratio of water insoluble polymer to enteric polymer may vary from 4:1 to 1:2, preferably the polymers are present at a ratio of about 1:1. The water insoluble polymer typically used is ethylcellulose.

Exemplary water insoluble polymers include ethylcellulose, polyvinyl acetate (Kollicoat SR#0D from BASF), neutral copolymers based on ethyl acrylate and methylmethacrylate, copolymers of acrylic and methacrylic acid esters with quaternary ammonium groups such as EUDRAGIT® NE, RS and RS30D, RL or RL30D and the like. Exemplary water soluble polymers include low molecular weight HPMC, HPC, methylcellulose, polyethylene glycol (PEG of molecular weight>3000) at a thickness ranging from 1 weight % up to 10 weight % depending on the solubility of the active in water and the solvent or latex suspension based coating formulation used. The water insoluble polymer to water soluble polymer may typically vary from 95:5 to 60:40, preferably from 80:20 to 65:35.

Preferably, the formulations are designed with release profiles to limit interference with restful sleep, wherein the formulation releases the medicine when the individual would normally be awakened by an urge to urinate. For example, consider an individual who begins sleeping at 11 PM and is normally awakened at 12:30 AM, 3:00 AM, and 6:00 AM to urinate. A delayed-release vehicle could deliver the medicine at 12:15 AM, thereby delaying the need to urinate for perhaps 2-3 hours.

The pharmaceutical composition may be administered daily or administered on an as needed basis. In certain embodiments, the pharmaceutical composition is administered to the subject prior to bedtime. In some embodiments, the pharmaceutical composition is administered immediately before bedtime. In some embodiments, the pharmaceutical composition is administered within about two hours before bedtime, preferably within about one hour before bedtime. In another embodiment, the pharmaceutical composition is administered about two hours before bedtime. In a further embodiment, the pharmaceutical composition is administered at least two hours before bedtime. In another embodiment, the pharmaceutical composition is administered about one hour before bedtime. In a further embodiment, the pharmaceutical composition is administered at least one hour before bedtime. In a still further embodiment, the pharmaceutical composition is administered less than one hour before bedtime. In still another embodiment, the pharmaceutical composition is administered immediately before bedtime. Preferably, the pharmaceutical composition is administered orally.

The appropriate dosage ("therapeutically effective amount") of the active agent(s) in the immediate-component or delayed-release component will depend, for example, the severity and course of the condition, the mode of administration, the bioavailability of the particular agent(s), the age and weight of the patient, the patient's clinical history and response to the active agent(s), discretion of the physician, *etc.*

As a general proposition, the therapeutically effective amount of the active agent(s) in the immediate-release component or the delayed-release component is administered in the range of about 100 µg/kg body weight/day to about 100 mg/kg body weight/day whether by one or more administrations. In some embodiments, the range of each active agent administered daily is from about 100 µg/kg body weight/day to about 50 mg/kg body weight/day, 100 µg/kg body weight/day to about 10 mg/kg body weight/day, 100 µg/kg body weight/day to about 1 mg/kg body weight/day, 100 µg/kg body weight/day to about 10 mg/kg body weight/day, 500 µg/kg body weight/day to about 100 mg/kg body weight/day, 500 µg/kg body weight/day to about 50 mg/kg body weight/day, 500 µg/kg body weight/ day to about 5 mg/kg body weight/ day, 1 mg/kg body weight/day to about 100 mg/kg body weight/day, 1 mg/kg body weight/day to about 50 mg/kg body weight/ day, 1 mg/kg body weight/day to about 10 mg/kg body weight/day, 5 mg/kg body weight/dose to about 100 mg/kg body weight/day, 5 mg/kg body weight/dose to about 50 mg/kg body weight/day, 10 mg/kg body weight/day to about 100 mg/kg body weight/day, and 10 mg/kg body weight/day to about 50 mg/kg body weight/day.

The active agent(s) described herein may be included in an immediate-release component or a delayed-release component or combinations thereof for daily oral administration at a single dose or combined dose range of 1 mg to 2000 mg, 5 mg to 2000 mg, 10 mg to 2000 mg, 50 mg to 2000 mg, 100 mg to 2000 mg, 200 mg to 2000 mg, 500 mg to 2000 mg, 5 mg to 1800 mg, 10 mg to 1600 mg, 50 mg to 1600 mg, 100 mg to 1500 mg, 150 mg to 1200 mg, 200 mg to 1000 mg, 300 mg to 800 mg, 325 mg to 500 mg, 1 mg to 1000 mg, 1 mg to 500 mg, 1 mg to 200 mg, 5 mg to 1000 mg, 5 mg to 500 mg, 5 mg to 200 mg, 10 mg to 1000 mg, 10 mg to 500 mg, 10 mg to 200 mg, 50 mg to 1000 mg, 50 mg to 500 mg, 50 mg to 200 mg, 100 mg to 250 mg, 100 mg to 500 mg, 250 mg to 1000 mg, 250 mg to 500 mg, 500 mg to 1000 mg, 500 mg to 2000 mg. As expected, the dosage will be dependent on the condition, size, age and condition of the patient.

In some embodiments, the pharmaceutical composition comprises a single analgesic agent. The single analgesic agent is acetaminophen.

The single analgesic agent is given at a daily dose of 5 mg to 2000 mg, 20 mg to 2000 mg, 5 mg to 1000 mg, 20 mg to 1000 mg, 50 mg to 500 mg, 100 mg to 500 mg, 250 mg to 500 mg, 250 mg to 1000 mg or 500 mg to 1000 mg. In certain embodiments, the pharmaceutical composition comprises acetaminophen as a single analgesic agent and the analgesic agent is administered orally at a daily dose in the range of 5 mg to 2000 mg, 20 mg to 2000 mg, 5 mg to 1000 mg, 20 mg to 1000 mg, 50 mg to 500 mg, 100 mg to 500 mg, 250 mg to 500 mg, 250 mg to 1000 mg or 500 mg to 1000 mg. In some embodiments, a second analgesic agent is given at a daily dose of 1 mg to 2000 mg, 5 mg to 2000 mg, 20 mg to 2000 mg, 5 mg to 1000 mg, 20 mg to 1000 mg, 50 mg to 500 mg, 100 mg to 500 mg, 250 mg to 500 mg, 250 mg to 1000 mg or 500 mg to 1000 mg.

In other embodiments, the pharmaceutical composition comprises a pair of analgesic agents. Examples of such paired analgesic agents include, but are not limited to, acetylsalicylic acid and acetaminophen, ibuprofen and acetaminophen, naproxen sodium and acetaminophen, nabumetone and acetaminophen, and acetaminophen and indomethancin. The paired analgesic agents are mixed at a weight ratio in the range of 0.1:1 to 10:1, 0.2:1 to 5:1 or 0.3:1 to 3:1, with a combined dose in the range of 5 mg to 2000 mg, 20 mg to 2000 mg, 100 mg to 2000 mg, 200 mg to 2000 mg, 500 mg to 2000 mg, 5 mg to 1500 mg, 20 mg to 1500 mg, 100 mg to 1500 mg, 200 mg to 1500 mg, 500 mg to 1500 mg, 5 mg to 1000 mg, 20 mg to 1000 mg, 100 mg to 1000 mg, 250 mg to 500 mg, 250 mg to 1000 mg, 250 mg to 1500 mg, 500 mg to 1000 mg, 500 mg to 1500 mg, 1000 mg to 1500 mg, and 1000 mg to 2000 mg. In one embodiment, the paired analgesic agents are mixed at a weight ratio of 1:1.

In some other embodiments, the pharmaceutical composition of the present application further comprises one or more antimuscarinic agents. Examples of the antimuscarinic agents include, but are not limited to, oxybutynin, solifenacin, darifenacin, fesoterodine, tolterodine, trospium and atropine. The daily dose of antimuscarinic agent is in the range of 0.01 mg to 100 mg, 0.1 mg to 100 mg, 1 mg to 100 mg, 10 mg to 100 mg, 0.01 mg to 25 mg, 0.1 mg to 25 mg, 1 mg to 25 mg, 10 mg to 25 mg, 0.01 mg to 10 mg, 0.1 mg to 10 mg, 1 mg to 10 mg, 10 mg to 100 mg and 10 mg to 25 mg.

Discussed herein is a method for reducing the frequency of urination by administering to a person in need thereof a pharmaceutical composition formulated in an immediate-release formulation. In some embodiments, the pharmaceutical composition comprises one or more analgesic agents and one or more antimuscarinic agents. In other embodiments, the pharmaceutical composition comprises one or more analgesic agents and one or more antidiuretic agents. In yet other embodiments, the pharmaceutical composition comprises one or more analgesic agents, one or more antimuscarinic agents, and one or more antidiuretic agents.

In other embodiments, the pharmaceutical composition of the present application further comprises one or more spasmolytics. Examples of spasmolytics include, but are not limited to, carisoprodol, benzodiazepines, baclofen, cyclobenzaprine, metaxalone, methocarbamol, clonidine, clonidine analog, and dantrolene. In some embodiments, the spasmolytics is used at a daily dose of 1 mg to 1000 mg, 1 mg to 100 mg, 10 mg to 1000 mg, 10 mg to 100 mg, 20 mg to 1000 mg, 20 mg to 800 mg, 20 mg to 500 mg, 20 mg to 200 mg, 50 mg to 1000 mg, 50 mg to 800 mg, 50 mg to 200 mg, 100 mg to 800 mg, 100 mg to 500 mg, 200 mg to 800 mg, and 200 mg to 500 mg. The spasmolytics may be formulated, alone or together with other active ingredient(s) in the pharmaceutical composition, for immediate-release, delayed-extended release or combinations thereof.

In certain embodiments, the pharmaceutical composition comprises two or more analgesic agents. In other embodiments, the pharmaceutical composition comprises one or more analgesic agents and one or more antimuscarinic agents and/or antidiuretic agents. The pharmaceutical composition may be formulated into a tablet, capsule, dragee, powder, granulate, liquid, gel or emulsion form. Said liquid, gel or emulsion may be ingested by the subject in naked form or contained within a capsule.

In certain embodiments, the antimuscarinic agent is selected from the group consisting of oxybutynin, solifenacin, darifenacin and atropine.

In some embodiments, the pharmaceutical composition comprises a single analgesic agent and a single antimuscarinic agent. The single analgesic agent is acetaminophen. The analgesic agent and anti-muscarinic agent may be given at doses in the ranges described above.

Discussed herein is a method for treating nocturia by administering to a subject in need thereof (1) one or more analgesic agents and (2) one or more antidiuretic agents. In certain arrangements, the antidiuretic agent(s) act to: (1) increase vasopressin secretion; (2) increase vasopressin receptor activation; (3) reduce secretion of atrial natriuretic peptide (ANP) or C-type natriuretic peptide (CNP); or (4) reduce ANP and/or CNP receptor activation.

Exemplary antidiuretic agents include, but are not limited to, antidiuretic hormone (ADH), angiotensin II, aldosterone, vasopressin, vasopressin analogs (*e.g*., desmopressin argipressin, lypressin, felypressin, ornipressin, terlipressin); vasopressin receptor agonists, atrial natriuretic peptide (ANP) and C-type natriuretic peptide (CNP) receptor (*i.e.,* NPR1, NPR2, NPR3) antagonists (*e.g.,* HS-142-1, isatin, [Asu7,23']b-ANP-(7-28)], anantin, a cyclic peptide from Streptomyces coerulescens, and 3G12 monoclonal antibody); somatostatin type 2 receptor antagonists (*e.g*., somatostatin), and pharmaceutically-acceptable derivatives, analogs, salts, hydrates, and solvates thereof.

In certain arrangements, the one or more analgesic agent and one or more antidiuretic agents are formulated for delayed-release.

Discussed herein is a method for treating nocturia by administering to a person in need thereof a first pharmaceutical composition comprising a diuretic, followed with a second pharmaceutical composition comprising one or more analgesic agents. The first pharmaceutical composition is dosed and formulated to have a diuretic effect within 6 hours of administration and is administered at least 8 hours prior to bedtime. The second pharmaceutical composition is administered within 2 hours prior to bedtime.

Examples of diuretics include, but are not limited to, acidifying salts, such as CaCl₂ and NH₄Cl; arginine vasopressin receptor 2 antagonists, such as amphotericin B and lithium citrate; aquaretics, such as Goldenrod and Junipe; Na-H exchanger antagonists, such as dopamine; carbonic anhydrase inhibitors, such as acetazolamide and dorzolamide; loop diuretics, such as bumetanide, ethacrynic acid, furosemide and torsemide; osmotic diuretics, such as glucose and mannitol; potassium-sparing diuretics, such as amiloride, spironolactone, triamterene, potassium canrenoate; thiazides, such as bendroflumethiazide and hydrochlorothiazide; and xanthines, such as caffeine, theophylline and theobromine.

In some arrangements, the second pharmaceutical composition further comprises one or more antimuscarinic agents. Examples of the antimuscarinic agents include, but are not limited to, oxybutynin, solifenacin, darifenacin, fesoterodine, tolterodine, trospium and atropine. The second pharmaceutical composition may be formulated in immediate-release formulation or delayed-release formulation. In one arrangement, the first pharmaceutical composition is formulated for immediate-release and the second pharmaceutical composition is formulated for delayed-release.

In some other arrangements, the second pharmaceutical composition further comprises one or more antidiuretic agents.

Discussed herein is a pharmaceutical composition comprising a plurality of active ingredients and a pharmaceutically acceptable carrier. In some arrangements, the plurality of active ingredients comprise two or more analgesics. In other arrangements, the plurality of active ingredients comprise one or more analgesics and one or more antimuscarinic agents. In other arrangements, the plurality of active ingredients comprise one or more analgesics and one or more antidiuretic agents. In yet other arrangements, the plurality of active ingredients comprise one or more analgesics, one or more antidiuretic agents, and one or more antimuscarinic agents. In other arrangements, at least one of said plurality of active ingredients is formulated for delayed-release.

In some arrangements, the pharmaceutical composition comprises two analgesics selected from the group consisting of cetylsalicylic acid, ibuprofen, naproxen sodium, nabumetone, acetaminophen and indomethancin. In other arrangements, the pharmaceutical composition comprises one or more analgesics selected from the group consisting of acetylsalicylic acid, ibuprofen, naproxen sodium, nabumetone, acetaminophen and indomethancin; and an antimuscarinic agent selected from the group consisting of oxybutynin, solifenacin, darifenacin and atropine.

As used herein, "pharmaceutically acceptable carrier" includes any and all solvents, dispersion media, coatings, antibacterial and antifungal agents, isotonic and absorption delaying agents, sweeteners and the like. The pharmaceutically acceptable carriers may be prepared from a wide range of materials including, but not limited to, flavoring agents, sweetening agents and miscellaneous materials such as buffers and absorbents that may be needed in order to prepare a particular therapeutic composition. The use of such media and agents with pharmaceutically active substances is well known in the art. Except insofar as any conventional media or agent is incompatible with the active ingredient, its use in the therapeutic compositions is contemplated.

Discussed herein is a method for reducing the frequency of urination by administering to a subject in need thereof, two or more analgesic agents alternatively to prevent the development of drug resistance. In one arrangement, the method comprises administering a first analgesic agent for a first period of time and then administering a second analgesic agent for a second period of time. In another arrangement, the method further comprises administering a third analgesic agent for a third period of time. The first, second and third analgesic agents are different from each other and may be selected from the group consisting of: salicylates, aspirin, salicylic acid, methyl salicylate, diflunisal, salsalate, olsalazine, sulfasalazine, para-aminophenol derivatives, acetanilide, acetaminophen, phenacetin, fenamates, mefenamic acid, meclofenamate, sodium meclofenamate, heteroaryl acetic acid derivatives, tolmetin, ketorolac, diclofenac, propionic acid derivatives, ibuprofen, naproxen sodium, naproxen, fenoprofen, ketoprofen, flurbiprofen, oxaprozin; enolic acids, oxicam derivatives, piroxicam, meloxicam, tenoxicam, ampiroxicam, droxicam, pivoxicam, pyrazolon derivatives, phenylbutazone, oxyphenbutazone, antipyrine, aminopyrine, dipyrone, coxibs, celecoxib, rofecoxib, nabumetone, apazone, nimesulide, indomethacin, sulindac, etodolac, and isobutylphenyl propionic acid.

In one arrangement, the first analgesic agent is acetaminophen, the second analgesic agent is ibuprofen and the third analgesic agent is naproxen sodium. The length of each period may vary depending on the subject's response to each analgesic agent. In some arrangements, each period lasts from 3 days to three weeks. In another arrangement, one or more of the first, second and third analgesic is formulated for delayed-release.

The present invention is further illustrated by the following example which should not be construed as limiting.

### EXAMPLE 1: INHIBITION OF THE URGE TO URINATE

Twenty volunteer subjects, both male and female were enrolled, each of which experienced premature urge or desire to urinate, interfering with their ability to sleep for a sufficient period of time to feel adequately rested. Each subject ingested 400-800 mg of ibuprofen as a single dose prior to bedtime. At least 14 subjects reported that they were able to rest better because they were not being awakened as frequently by the urge to urinate.

Several subjects reported that after several weeks of nightly use of ibuprofen, the benefit of less frequent urges to urinate was no longer being realized. However, all of these subjects further reported the return of the benefit after several days of abstaining from taking the dosages.

### EXAMPLE 2: EFFECT OF ANALGESIC AGENTS, BOTULINUM NEUROTOXIN AND ANTIMUSCARINIC AGENTS ON MACROPHAGE RESPONSES TO INFLAMMATORY AND NON-INFLAMMATORY STIMULI

### Experimental Design

This study is designed to determine the dose and *in vitro* efficacy of analgesics and antimuscarinic agents in controlling macrophage response to inflammatory and non-inflammatory stimuli mediated by Cox-2 and prostaglandins (PGE, PGH, *etc*.). It establishes baseline (dose and kinetic) responses to inflammatory and non-inflammatory effectors in bladder cells. Briefly, cultured cells are exposed to analgesic agents and/or antimuscarinic agents in the absence or presence of various effectors.

The effectors include: lipopolysaccharide (LPS), an inflammatory agent and COX2 inducer, as inflammatory stimuli; carbachol or acetylcholine, a stimulator of smooth muscle contraction, as non-inflammatory stimuli; botulinum neurotoxin A, a known inhibitor of acetylcholine release, as positive control; and arachidonic acid (AA), gamma linolenic acid (DGLA) or eicosapentaenoic acid (EPA) as precursors of prostaglandins, which are produced following the sequential oxidation of AA, DGLA or EPA inside the cell by cyclooxygenases (COX1 and COX2) and terminal prostaglandin synthases.

The analgesic agents include: Salicylates such as aspirin, iso-butyl-propanoic-phenolic acid derivative (ibuprofen) such as Advil, Motrin, Nuprin, and Medipren, naproxen sodium such as Aleve, Anaprox, Antalgin, Feminax Ultra, Flanax, Inza, Midol Extended Relief, Nalgesin, Naposin, Naprelan, Naprogesic, Naprosyn, Naprosyn suspension, EC-Naprosyn, Narocin, Proxen, Synflex and Xenobid, acetic acid derivative such as indomethacin (Indocin),1-naphthaleneacetic acid derivative such as nabumetone or relafen, N-acetyl-para-aminophenol (APAP) derivative such as acetaminophen or paracetamol

(Tylenol) and Celecoxib.

The antimuscarinic agents include: oxybutynin, solifenacin, darifenacin and atropine.

Macrophages are subjected to short term (1-2 hrs) or long term (24-48 hrs) stimulation of with:
1) Each analgesic agent alone at various doses.
(2) Each analgesic agent at various doses in the presence of LPS.
(3) Each analgesic agent at various doses in the presence of carbachol or acetylcholine.
(4) Each analgesic agent at various doses in the presence of AA, DGLA, or EPA.
(5) Botulinum neurotoxin A alone at various doses.
(6) Botulinum neurotoxin A at various doses in the presence of LPS.
(7) Botulinum neurotoxin A at various doses in the presence of carbachol or acetylcholine.
(8) Botulinum neurotoxin A at various doses in the presence of AA, DGLA, or EPA.
(9) Each antimuscarinic agent alone at various doses.
(10) Each antimuscarinic agent at various doses in the presence of LPS.
(11) Each antimuscarinic agent at various doses in the presence of carbachol or acetylcholine.
(12) Each antimuscarinic agent at various doses in the presence of AA, DGLA, or EPA.

The cells are then analyzed for the release of PGH₂, PGE, PGE₂, Prostacydin, Thromboxane, IL-1β, IL-6, TNF-α, the COX2 activity, the production of cAMP and cGMP, the production of IL-1β, IL-6, TNF-α and COX2 mRNA, and surface expression of CD80, CD86 and MHC class II molecules.

### Materials and Methods

### Macrophage cells

Murine RAW264.7 or J774 macrophage cells (obtained from ATCC) were used in this study. Cells were maintained in a culture medium containing RPMI 1640 supplemented with 10 % fetal bovine serum (FBS), 15 mM HEPES, 2 mM L-glutamine, 100 U/ml penicillin, and 100 µg / ml of streptomycin. Cells were cultured at 37° C in a 5 % CO₂ atmosphere and split (passages) once a week.

### In vitro treatment of macrophage cells with analgesics

RAW264.7 macrophage cells were seeded in 96-well plates at a cell density of 1.5x10⁵ cells per well in 100 µl of the culture medium. The cells were treated with (1) various concentrations of analgesic (acetaminophen, aspirin, ibuprophen or naproxen), (2) various concentrations of lipopolysaccharide (LPS), which is an effector of inflammatory stimuli to macrophage cells, (3) various concentrations of carbachol or acetylcholine, which are effectors of non-inflammatory stimuli, (4) analgesic and LPS or (5) analgesic and carbachol or acetylcholine. Briefly, the analgesics were dissolved in FBS-free culture medium (*i.e.,* RPMI 1640 supplemented with 15 mM HEPES, 2 mM L-glutamine, 100 U / ml penicillin, and 100 µg / ml of streptomycin), and diluted to desired concentrations by serial dilution with the same medium. For cells treated with analgesic in the absence of LPS, 50 µl of analgesic solution and 50 µl of FBS-free culture medium were added to each well. For cells treated with analgesic in the presence of LPS, 50 µl of analgesic solution and 50 µl of LPS (from *Salmonella typhimurium*) in FBS-free culture medium were added to each well. All conditions were tested in duplicates.

After 24 or 48 hours of culture, 150 µl of culture supernatants were collected, spun down for 2 min at 8,000 rpm at 4°C to remove cells and debris and stored at -70°C for analysis of cytokine responses by ELISA. The cells were collected and washed by centrifugation (5 min at 1,500 rpm at 4°C) in 500 µl of Phosphate buffer (PBS). Half of the cells were then snap frozen in liquid nitrogen and stored at -70°C. The remaining cells were stained with fluorescent monoclonal antibodies and analyzed by flow cytometry.

### Flow cytometry analysis of co-stimulatory molecule expression

For flow cytometry analysis, macrophages were diluted in 100 µl of FACS buffer (phosphate buffered saline (PBS) with 2% bovine serum albumin (BSA) and 0.01% NaN₃) and stained 30 min at 4°C by addition of FITC-conjugated anti-CD40, PE-conjugated anti-CD80, PE-conjugated anti-CD86 antibody, anti MHC class II (I-A^{d}) PE (BD Bioscience). Cells were then washed by centrifugation (5 min at 1,500 rpm at 4°C) in 300 µl of FACS buffer. After a second wash, cells were re-suspended in 200 µl of FACS buffer and the percentage of cells expressing a given marker (single positive), or a combination of markers (double positive) were analyzed with the aid of an Accuri C6 flow cytometer (BD Biosciences).

### Analysis of cytokine responses by ELISA

Culture supernatants were subjected to cytokine-specific ELISA to determine IL-1β, IL-6 and TNF-α responses in cultures of macrophages treated with analgesic, LPS alone or a combination of LPS and analgesic. The assays were performed on Nunc MaxiSorp Immunoplates (Nunc) coated overnight with 100 µl of anti-mouse IL-6, TNF-α mAbs (BD Biosciences) or IL-1β mAb (R&D Systems) in 0.1 M sodium bicarbonate buffer (pH 9.5). After two washes with PBS (200 µl per well), 200 µl of PBS 3% BSA were added in each well (blocking) and the plates incubated for 2 hours at room temperature. Plates were washed again two times by addition of 200 µl per well, 100 µl of cytokine standards and serial dilutions of culture supernatants were added in duplicate and the plates were incubated overnight at 4°C. Finally, the plates were washed twice and incubated with 100 µl of secondary biotinylated anti-mouse IL-6, TNFα mAbs (BD Biosciences) or IL-1β (R&D Systems) followed by peroxidase-labelled goat anti-biotin mAb (Vector Laboratories). The colorimetric reaction was developed by the addition of 2,2'-azino-bis (3)-ethylbenzylthiazoline-6-sulfonic acid (ABTS) substrate and H₂0₂ (Sigma) and the absorbance measured at 415 nm with a Victor® V multilabel plate reader (PerkinElmer).

### Determination of COX2 activity and the production of cAMP and cGMP

The COX2 activity in the cultured macrophages is determined by COX2 activity assay. The production of cAMP and cGMP is determined by the cAMP assay and cGMP assay. These assays are performed routinely in the art.

### Results

Table 1 summarizes the experiments performed with Raw 264 macrophage cell line and main findings in terms of the effects of analgesics on cell surface expression of costimulatory molecules CD40 and CD80. Expression of these molecules is stimulated by COX2 and inflammatory signals and thus, was evaluated to determine functional consequences of inhibition of COX2.

As shown in Table 2, acetaminophen, aspirin, ibuprophen and naproxen inhibit basal expression of co-stimulatory molecules CD40 and CD80 by macrophages at all the tested doses (*i.e.,* 5x 10⁵ nM, 5x 10⁴ nM, 5x 10³ nM, 5x 10² nM, 50 nM and 5 nM), except for the highest dose (*i.e.,* 5x 10⁶ nM), which appears to enhance, rather than inhibit, expression of the co-stimulatory molecules. As shown in Figures 1A and 1B, such inhibitory effect on CD40 and CD50 expression was observed at analgesic doses as low as 0.05 nM (*i.e.,* 0.00005 µM). This finding supports the notion that a controlled release of small doses of analgesic may be preferable to acute delivery of large doses. The experiment also revealed that acetaminophen, aspirin, ibuprophen and naproxen have a similar inhibitory effect on LPS induced expression of CD40 and CD80.

**Table 1. Summary of experiments**

| | Control | LPS *Salmonella typhimurium* | Acetaminophen | Aspirin | Ibuprophen | Naproxen |
|---|---|---|---|---|---|---|
| TESTS | | | | | | |
| 1 | X | | | | | |
| 2 | X | Dose responses (0, 5, 50, 1000) ng/mL | | | | |
| 3 | X | | Dose responses | | | |
| | | | (0, 5, 50, 500, 5x10³, 5x10⁴, 5x10⁵, 5x10⁶) nM | | | |
| 4 | X | X (5 ng/mL) | Dose responses | | | |
| | | X (50 ng/mL | (0, 5, 50, 500, 5.10³, 5.10⁴, 5.10⁵, 5.10⁶) nM | | | |
| | | X (1000 ng/mL) | | | | |

| ANALYSIS | | | | | | |
|---|---|---|---|---|---|---|
| a | Characterization of activation/stimulatory status: Flow cytometry analysis of CD40, CD80, CD86 and MHC class II | | | | | |
| b | Mediators of inflammatory responses: ELISA analysis of IL-1β, IL-6, TNF-α | | | | | |

**Table 2. Summary of main findings**

| Effectors | % Positive | Negative Control | LPS 5 ng/ml | Dose analgesic (nM) | | | | | | |
|---|---|---|---|---|---|---|---|---|---|---|
| | | | | 5.10⁶ | 5.10⁵ | 5.10⁴ | 5.10³ | 500 | 50 | 5 |
| | CD40⁺CD80⁺ | 20.6 | 77.8 | | | | | | | |
| Acetaminophen | CD40⁺CD80⁺ | | | 63 | 18 | 12 | 9.8 | 8.3 | 9.5 | 7.5 |
| Aspirin | CD40⁺CD80⁺ | | | 44 | 11 | 10.3 | 8.3 | 8 | 10.5 | 7.5 |
| Ibuprophen | CD40⁺CD80⁺ | | | ND* | 6.4 | 7.7 | 7.9 | 6.0 | 4.9 | 5.8 |
| Naproxen | CD40⁺CD80⁺ | | | 37 | 9.6 | 7.7 | 6.9 | 7.2 | 6.8 | 5.2 |

| | | | | Analgesic plus LPS | | | | | | |
|---|---|---|---|---|---|---|---|---|---|---|
| Acetaminophen | CD40⁺CD80⁺ | | | 95.1 | 82.7 | 72.4 | 68.8 | 66.8 | 66.2 | 62.1 |
| Aspirin | CD40⁺CD80⁺ | | | 84.5 | 80 | 78.7 | 74.7 | 75.8 | 70.1 | 65.7 |
| Ibuprophen | CD40⁺CD80⁺ | | | ND | 67 | 77.9 | 72.9 | 71.1 | 63.7 | 60.3 |
| Naproxen | CD40⁺CD80⁺ | | | 66.0 | 74.1 | 77.1 | 71.0 | 68.8 | 72 | 73 |

| | | | | | | | | | | |
|---|---|---|---|---|---|---|---|---|---|---|
| * ND: not done (toxicity) | | | | | | | | | | |

Table 3 summarizes the results of several studies that measured serum levels of analgesic after oral therapeutic doses in adult humans. As shown in Table 3, the maximum serum levels of analgesic after an oral therapeutic dose are in the range of 10⁴ to 10⁵ nM. Therefore, the doses of analgesic tested *in vitro* in Table 2 cover the range of concentrations achievable *in vivo* in human.

**Table 3. Serum levels of analgesic in human blood after oral therapeutic doses**

| Analgesic drug | Molecular weight | Maximum serum levels after oral therapeutic doses | | References |
|---|---|---|---|---|
| | | mg/L | nM | |
| **Acetaminophen** (Tylenol) | 151.16 | 11-18 | 7.2x10⁴-1.19x10⁵ | * BMC Clinical Pharmacology.2010, 10:10 |
| | | | | * Anaesth Intensive Care. 2011, 39:242 |
| **Aspirin** (Acetylsalicylic acid) | 181.66 | 30-100 | 1.65x10⁵-5.5x10⁵ | * Disposition of Toxic Drugs and Chemicals in Man, 8th Edition, Biomedical Public, Foster City, CA, 2008, pp. 22-25 |
| | | | | |
| | | | | * J Lab Clin Med. 1984 Jun;103:869 |
| **Ibuprofen** (Advil, Motrin) | 206.29 | 24-32 | 1.16x10⁵-1.55 x10⁵ | * BMC Clinical Pharmacology2010, 10:10 |
| | | | | * J Clin Pharmacol. 2001, 41:330 |
| **Naproxen** (Aleve) | 230.26 | Up to 60 | Up to 2.6x10⁵ | * J Clin Pharmacol. 2001, 41:330 |

### EXAMPLE 3: EFFECT OF ANALGESIC AGENTS, BOTULINUM NEUROTOXIN AND ANTIMUSCARINIC AGENTS ON BLADDER SMOOTH MUSCLE CELL RESPONSES TO INFLAMMATORY AND NON-INFLAMMATORY STIMULI

### Experimental Design

This study is designed to characterize how the optimal doses of analgesic determined in Example 2 affect bladder smooth muscle cells in cell culture or tissue cultures, and to address whether different classes of analgesics can synergize to more efficiently inhibit COX2 and PGE2 responses.

The effectors, analgesic agents and antimuscarinic agents are described in Example 2.

Primary culture of mouse bladder smooth muscle cells are subjected to short term (1-2 hrs) or long term (24-48 hrs) stimulation of with:
(1) Each analgesic agent alone at various doses.
(2) Each analgesic agent at various doses in the presence of LPS.
(3) Each analgesic agent at various doses in the presence of carbachol or acetylcholine.
(4) Each analgesic agent at various doses in the presence of AA, DGLA, or EPA.
(5) Botulinum neurotoxin A alone at various doses.
(6) Botulinum neurotoxin A at various doses in the presence of LPS.
(7) Botulinum neurotoxin A at various doses in the presence of carbachol or acetylcholine.
(8) Botulinum neurotoxin A at various doses in the presence of AA, DGLA, or EPA.
(9) Each antimuscarinic agent alone at various doses.
(10) Each antimuscarinic agent at various doses in the presence of LPS.
(11) Each antimuscarinic agent at various doses in the presence of carbachol or acetylcholine.
(12) Each antimuscarinic agent at various doses in the presence of AA, DGLA, or EPA.

The cells are then analyzed for the release of PGH₂, PGE, PGE₂, Prostacydin, Thromboxane, IL-1β, IL-6, TNF-α, the COX2 activity, the production of cAMP and cGMP, the production of IL-1β, IL-6, TNF-α and COX2 mRNA, and surface expression of CD80, CD86 and MHC class II molecules.

### Materials and Methods

### Isolation and purification of murine bladder cells

Bladder cells were removed from euthanized animals C57BL/6 mice (8-12 weeks old) and cells were isolated by enzymatic digestion followed by purification on a Percoll gradient. Briefly, bladders from 10 mice were minced with scissors to fine slurry in 10 ml of digestion buffer (RPMI 1640, 2% fetal bovine serum, 0.5 mg/ml collagenase, 30 µg/ml DNase). Bladder slurries were enzymatically digested for 30 minutes at 37°C. Undigested fragments were further dispersed through a cell-trainer. The cell suspension was pelleted and added to a discontinue 20%, 40% and 75% Percoll gradient for purification on mononuclear cells. Each experiment used 50-60 bladders.

After washes in RPMI 1640, bladder cells were resuspended RPMI 1640 supplemented with 10 % fetal bovine serum, 15 mM HEPES, 2 mM L-glutamine, 100 U/ml penicillin, and 100 µg / ml of streptomycin and seeded in clear-bottom black 96-well cell culture microculture plates at a cell density of 3x10⁴ cells per well in 100 µl. Cells were cultured at 37° C in a 5 % CO₂ atmosphere.

### In vitro treatment of cells with analgesics

Bladder cells were treated with analgesic solutions (50 µl/well) either alone or together with carbachol (10-Molar, 50 µl/well), as an example of non-inflammatory stimuli, or lipopolysaccharide (LPS) of *Salmonella typhimurium* (1 µg/ml, 50 µl/well), as an example of non-inflammatory stimuli. When no other effectors were added to the cells, 50 µl of RPMI 1640 without fetal bovine serum were added to the wells to adjust the final volume to 200 µl.

After 24 hours of culture, 150 µl of culture supernatants were collected, spun down for 2 min at 8,000 rpm at 4°C to remove cells and debris and stored at -70°C for analysis of Prostaglandin E2 (PGE₂) responses by ELISA. Cells were fixed, permeabilized and blocked for detection of Cyclooxygenase-2 (COX2) using a fluorogenic substrate. In selected experiment cells were stimulated 12 hours in vitro for analysis of COX2 responses.

### Analysis of COX2 responses

COX2 responses were analyzed by a Cell-Based ELISA using Human/mouse total COX2 immunoassay (R&D Systems), following the instructions of the manufacturer. Briefly, after cells fixation and permeabilization, a mouse anti-total COX2 and a rabbit anti-total GAPDH were added to the wells of the clear-bottom black 96-well cell culture microculture plates. After incubation and washes, an HRP-conjugated anti-mouse IgG and an AP-conjugated anti-rabbit IgG were added to the wells. Following another incubation and set of washes, the HRP- and AP-fluorogenic substrates were added. Finally, a Victor® V multilabel plate reader (PerkinElmer) was used to read the fluorescence emitted at 600 nm (COX2 fluorescence) and 450 nm (GAPDH fluorescence). Results are expressed as relative levels of total COX2 as determined by relative fluorescence unit (RFUs) and normalized to the housekeeping protein GAPDH.

### Analysis of PGE2 responses

Prostaglandin E2 responses were analyzed by a sequential competitive ELISA (R&D Systems). More specifically, culture supernatants or PGE2 standards were added to the wells of a 96-well polystyrene microplate coated with a goat anti-mouse polyclonal antibody. After one hour incubation on a microplate shaker, an HRP-conjugated PGE2 was added and plates incubated for an additional two hours at room temperature. The plates were then washed and HRP substrate solution added to each well. The color was allowed to develop for 30 min and the reaction stopped by addition sulfuric acid before reading the plate at 450 nm with wavelength correction at 570 nm. Results are expressed as mean pg/ml of PGE2.

### Other assays

The release of PGH₂, PGE, Prostacydin, Thromboxane, IL-1β, IL-6, and TNF-α, the production of cAMP and cGMP, the production of IL-1β, IL-6, TNF-α and COX2 mRNA, and surface expression of CD80, CD86 and MHC class II molecules are determined as described in Example 2.

### Results

### Analgesics inhibit COX2 responses of murine bladder cells to an inflammatory stimuli

Several analgesics (acetaminophen, aspirin, ibuprofen and naproxen) were tested on mouse bladder cells at the concentration of 5 µM or 50 µM to determine whether the analgesics could induce COX2 responses. Analysis of 24-hour cultures showed that none of the analgesics tested induced COX2 responses in murine bladder cells *in vitro.*

The effect of these analgesics on the COX2 responses of murine bladder cells to carbachol or LPS stimulation *in vitro* was also tested. As indicated in Table 1, the dose of carbachol tested has no significant effect on COX2 levels in murine bladder cells. On the other hand, LPS significantly increased total COX2 levels. Interestingly, acetaminophen, aspirin, ibuprofen and naproxen could all suppress the effect of LPS on COX2 levels. The suppressive effect of the analgesic was seen when these drugs were tested at either 5 µM or 50 µM (Table 4).

**Table 4. COX2 expression by murine bladder cells after in vitro stimulation and treatment with analgesics**

| **Stimuli** | **Analgesic** | **Total COX2 levels (Normalized RFUs)** |
|---|---|---|
| None | None | 158 ± 18 |
| Carbachol (mM) | None | 149 ± 21 |
| LPS (1µg/ml) | None | 420 ± 26 |
| LPS (1µg/ml) | Acetaminophen (5 µM) | 275 ± 12 |
| LPS (1µg/ml) | Aspirin (5 µM) | 240 ± 17 |
| LPS (1µg/ml) | Ibuprofen (5 µM)) | 253 ± 32 |
| LPS (1µg/ml) | Naproxen (5 µM) | 284 ± 11 |
| LPS (1µg/ml) | Acetaminophen (50 µM) | 243 ± 15 |
| LPS (1µg/ml) | Aspirin (50 µM) | 258 ± 21 |
| LPS (1µg/ml) | Ibuprofen (50 µM) | 266 ± 19 |
| LPS (1µg/ml) | Naproxen (50 µM) | 279 ± 23 |

### Analgesics inhibit PGE2 responses of murine bladder cells to an inflammatory stimuli

The secretion of PGE2 in culture supernatants of murine bladder cells was measured to determine the biological significance of the alteration of murine bladder cell COX2 levels by analgesics. As shown in Table 5, PGE2 was not detected in the culture supernatants of unstimulated bladder cells or bladder cells cultured in the presence of carbachol. Consistent with COX2 responses described above, stimulation of murine bladder cells with LPS induced the secretion of high levels of PGE2. Addition of the analgesics acetaminophen, aspirin, ibuprofen and naproxen suppressed the effect of LPS on PGE2 secretion and no difference was seen between the responses of cells treated with the 5 or 50 µM dose of analgesic.

**Table 5. PGE2 secretion by murine bladder cells after in vitro stimulation and treatment with analgesics**

| **Stimuli** | **Analgesic** | **PGE2 levels (pg/ml)** |
|---|---|---|
| None | None | < 20.5 |
| Carbachol (mM) | None | < 20.5 |
| LPS (1µg/ml) | None | 925 ± 55 |
| LPS (1µg/ml) | Acetaminophen (5 µM) | 619 ± 32 |
| LPS (1µg/ml) | Aspirin (5 µM) | 588 ± 21 |
| LPS (1µg/ml) | Ibuprofen (5 µM)) | 593 ± 46 |
| LPS (1µg/ml) | Naproxen (5 µM) | 597± 19 |
| LPS (1µg/ml) | Acetaminophen (50 µM) | 600 ± 45 |
| LPS (1µg/ml) | Aspirin (50 µM) | 571 ± 53 |
| LPS (1µg/ml) | Ibuprofen (50 µM) | 568 ± 32 |
| LPS (1µg/ml) | Naproxen (50 µM) | 588 ± 37 |

In summary, these data show that the analgesics alone at 5 µM or 50 µM do not induce COX2 and PGE2 responses in murine bladder cells. The analgesics at 5 µM or 50 µM, however, significantly inhibit COX2 and PGE2 responses of murine bladder cells stimulated *in vitro* with LPS (1 µg/ml). No significant effect of analgesics was observed on COX2 and PGE2 responses of murine bladder cells stimulated with carbachol (1 mM).

### EXAMPLE 4: EFFECT OF ANALGESIC AGENTS, BOTULINUM NEUROTOXIN AND ANTIMUSCARINIC AGENTS ON BLADDER SMOOTH MUSCLE CELL CONTRACTION.

### Experimental Design

Cultured mouse or rat bladder smooth muscle cells and mouse or rat bladder smooth muscle tissue are exposed to inflammatory stimuli and non-inflammatory stimuli in the presence of analgesic agent and/or antimuscarinic agent at various concentrations. The stimuli-induced muscle contraction is measured to evaluate the inhibitory effect of the analgesic agent and/or antimuscarinic agent.

The effectors, analgesic agents and antimuscarinic agents are described in Example 2.

Primary culture of mouse bladder smooth muscle cells are subjected to short term (1-2 hrs) or long term (24-48 hrs) stimulation of with:
(1) Each analgesic agent alone at various doses.
(2) Each analgesic agent at various doses in the presence of LPS.
(3) Each analgesic agent at various doses in the presence of carbachol or acetylcholine.
(4) Each analgesic agent at various doses in the presence of AA, DGLA, or EPA.
(5) Botulinum neurotoxin A alone at various doses.
(6) Botulinum neurotoxin A at various doses in the presence of LPS.
(7) Botulinum neurotoxin A at various doses in the presence of carbachol or acetylcholine.
(8) Botulinum neurotoxin A at various doses in the presence of AA, DGLA, or EPA.
(9) Each antimuscarinic agent alone at various doses.
(10) Each antimuscarinic agent at various doses in the presence of LPS.
(11) Each antimuscarinic agent at various doses in the presence of carbachol or acetylcholine.
(12) Each antimuscarinic agent at various doses in the presence of AA, DGLA, or EPA.

### Materials and Methods

Primary mouse bladder cells are isolated as described in Example 3. In selected experiments, cultures of bladder tissue are used. Bladder smooth muscle cell contractions are recorded with a Grass polygraph (Quincy Mass, USA).

### EXAMPLE 5: EFFECT OF ORAL ANALGESIC AGENTS AND ANTIMUSCARINIC AGENTS ON COX2 AND PGE2 RESPONSES OF BLADDER SMOOTH MUSCLE CELLS.

### Experimental design:

Normal mice and mice with over active bladder syndrome are given oral doses of aspirin, naproxen sodium, Ibuprofen, Indocin, nabumetone, Tylenol, Celecoxib, oxybutynin, solifenacin, darifenacin, atropine and combinations thereof. Control groups include untreated normal mice and untreated OAB mice without over active bladder syndrome. Thirty (30) min after the last doses, the bladders are collected and stimulated *ex vivo* with carbachol or acetylcholine. In selected experiments the bladders are treated with botulinum neurotoxin A before stimulation with carbachol. Animals are maintained in metabolic cages and frequency (and volume) of urination are evaluated. Bladder outputs are determined by monitoring water intake and cage litter weight. Serum PGH₂, PGE, PGE₂, Prostacydin, Thromboxane, IL-1β, IL-6, TNF-α, cAMP, and cGMP levels are determined by ELISA. CD80, CD86, MHC class II expression in whole blood cells are determined by flow cytometry.

At the end of the experiment, animals are euthanized and *ex vivo* bladder contractions are recorded with a Grass polygraph. Portions of bladders are fixed in formalin, and COX2 responses are analyzed by immunohistochemistry.

### EXAMPLE 6: EFFECT OF ANALGESIC AGENTS, BOTULINUM NEUROTOXIN AND ANTIMUSCARINIC AGENTS ON HUMAN BLADDER SMOOTH MUSCLE CELL RESPONSES TO INFLAMMATORY AND NON-INFLAMMATORY STIMULI

### Experimental Design

This study is designed to characterize how the optimal doses of analgesics determined in Examples 1-5 affect human bladder smooth muscle cells in cell culture or tissue cultures, and to address whether different classes of analgesics can synergize to more efficiently inhibit COX2 and PGE2 responses.

The effectors, analgesic agents and antimuscarinic agents are described in Example 2.

Human bladder smooth muscle cells are subjected to short term (1-2 hrs) or long term (24-48 hrs) stimulation of with:
(1) Each analgesic agent alone at various doses.
(2) Each analgesic agent at various doses in the presence of LPS.
(3) Each analgesic agent at various doses in the presence of carbachol or acetylcholine.
(4) Each analgesic agent at various doses in the presence of AA, DGLA, or EPA.
(5) Botulinum neurotoxin A alone at various doses.
(6) Botulinum neurotoxin A at various doses in the presence of LPS.
(7) Botulinum neurotoxin A at various doses in the presence of carbachol or acetylcholine.
(8) Botulinum neurotoxin A at various doses in the presence of AA, DGLA, or EPA.
(9) Each antimuscarinic agent alone at various doses.
(10) Each antimuscarinic agent at various doses in the presence of LPS.
(11) Each antimuscarinic agent at various doses in the presence of carbachol or acetylcholine.
(12) Each antimuscarinic agent at various doses in the presence of AA, DGLA, or EPA.

The cells are then analyzed for the release of PGH₂, PGE, PGE₂, Prostacydin, Thromboxane, IL-1β, IL-6, TNF-α, the COX2 activity, the production of cAMP and cGMP, the production of IL-1β, IL-6, TNF-α and COX2 mRNA, and surface expression of CD80, CD86 and MHC class II molecules.

### EXAMPLE 7: EFFECT OF ANALGESIC AGENTS, BOTULINUM NEUROTOXIN AND ANTIMUSCARINIC AGENTS ON HUMAN BLADDER SMOOTH MUSCLE CELL CONTRACTION.

### Experimental Design

Cultured human bladder smooth muscle cells are exposed to inflammatory stimuli and non-inflammatory stimuli in the presence of analgesic agent and/or antimuscarinic agent at various concentrations. The stimuli-induced muscle contraction is measured to evaluate the inhibitory effect of the analgesic agent and/or antimuscarinic agent.

The effectors, analgesic agents and antimuscarinic agents are described in Example 2.

Human bladder smooth muscle cells are subjected to short term (1-2 hrs) or long term (24-48 hrs) stimulation of with:
(1) Each analgesic agent alone at various doses.
(2) Each analgesic agent at various doses in the presence of LPS.
(3) Each analgesic agent at various doses in the presence of carbachol or acetylcholine.
(4) Each analgesic agent at various doses in the presence of AA, DGLA, or EPA.
(5) Botulinum neurotoxin A alone at various doses.
(6) Botulinum neurotoxin A at various doses in the presence of LPS.
(7) Botulinum neurotoxin A at various doses in the presence of carbachol or acetylcholine.
(8) Botulinum neurotoxin A at various doses in the presence of AA, DGLA, or EPA.
(9) Each antimuscarinic agent alone at various doses.
(10) Each antimuscarinic agent at various doses in the presence of LPS.
(11) Each antimuscarinic agent at various doses in the presence of carbachol or acetylcholine.
(12) Each antimuscarinic agent at various doses in the presence of AA, DGLA, or EPA.

Bladder smooth muscle cell contractions are recorded with a Grass polygraph (Quincy Mass, USA).

### EXAMPLE 8: EFFECT OF ANALGESIC AGENTS ON NORMAL HUMAN BLADDER SMOOTH MUSCLE CELL RESPONSES TO INFLAMMATORY AND NON INFLAMMATORY SIGNALS

### EXPERIMENTAL DESIGN

### Culture of normal human bladder smooth muscle cells

Normal human bladder smooth muscle cells were isolated by enzymatic digestion from macroscopically normal pieces of human bladder. Cells were expended *in vitro* by culture at 37° C in a 5 % CO₂ atmosphere in RPMI 1640 supplemented with 10 % fetal bovine serum, 15 mM HEPES, 2 mM L-glutamine, 100 U/ml penicillin, and 100 mg / ml of streptomycin and passage once a week by treatment with trypsin to detach cells followed by reseeding in a new culture flask. The first week of culture, the culture medium was supplemented with 0.5 ng/ml epidermal growth factor, 2 ng/ml fibroblast growth factor, and 5 µg/ml insulin.

### Treatment of normal human bladder smooth muscle cells with analgesics in vitro

Bladder smooth muscle cells trypsinized and seeded in microculture plates at a cell density of 3x10⁴ cells per well in 100 µl were treated with analgesic solutions (50 µl/ well) either alone or together carbachol (10-Molar, 50 µl/ well), as an example of non-inflammatory stimuli, or lipopolysaccharide (LPS) of *Salmonella typhimurium* (1 µg/ml, 50 µl/ well), as an example of non-inflammatory stimuli. When no other effectors were added to the cells, 50 µl of RPMI 1640 without fetal bovine serum were added to the wells to adjust the final volume to 200 µl.

After 24 hours of culture, 150 µl of culture supernatants were collected, spun down for 2 min at 8,000 rpm at 4°C to remove cells and debris and stored at -70°C for analysis of Prostaglandin E2 (PGE₂) responses by ELISA. Cells were fixed, permeabilized and blocked for detection of COX2 using a fluorogenic substrate. In selected experiments cells were stimulated 12 hours *in vitro* for analysis of COX2, PGE2 and cytokine responses.

### Analysis of COX2, PGE2 and cytokine responses

COX2 and PGE2 responses were analyzed as described in Example 3.

### Cytokine responses were analyzed as described in Example 2

### RESULTS

*Analgesics inhibit COX2 responses of normal human bladder smooth muscle cells to inflammatory and non*-*inflammatory stimuli* - Analysis of cells and culture supernatants after 24 hours of cultures showed that none of the analgesics tested alone induced COX2 responses in normal human bladder smooth muscle cells. However, as summarized in Table 6, carbachol induced low, but significant COX2 responses in normal human bladder smooth muscle cells. On the other hand, LPS treatment resulted in higher levels of COX2 responses in normal human bladder smooth muscle cells. Acetaminophen, aspirin, ibuprofen and naproxen could all suppress the effect of carbachol and LPS on COX2 levels. The suppressive effect of the analgesics was seen on LPS-induced responses when these drugs were tested at either 5 µM or 50 µM.

**Table 6. COX2 expression by normal human bladder smooth muscle cells after in vitro stimulation with inflammatory and non- inflammatory stimuli and treatment with analgesic**

| **Stimuli** | **Analgesic** | **Total COX2 levels^{#} (Normalized RFUs)** | **Total COX2 levels (Normalized RFUs)** |
|---|---|---|---|
| | | **subject 1** | **subject 2** |
| None | None | 230 | 199 |
| Carbachol 10⁻³ M | Acetaminophen (50 µM) | 437 | 462 |
| Carbachol 10⁻³ M | Aspirin (50 µM) | 298 | 310 |
| Carbachol 10⁻³ M | Ibuprofen (50 µM) | 312 | 297 |
| Carbachol 10⁻³ M | Naproxen (50 µM) | 309 | 330 |
| Carbachol 10⁻³ M | Acetaminophen (50 µM) | 296 | 354 |
| | | | |
| LPS (10 µg/ml) | None | 672 | 633 |
| LPS (10 µg/ml) | Acetaminophen (5 µM) | 428 | 457 |
| LPS (10 µg/ml) | Aspirin (5 µM) | 472 | 491 |
| LPS (10 µg/ml) | Ibuprofen (5 µM) | 417 | 456 |
| LPS (10 µg/ml) | Naproxen (5 µM | 458 | 501 |
| LPS (10 µg/ml) | Acetaminophen (50 µM) | 399 | 509 |
| LPS (10 µg/ml) | Aspirin (50 µM) | 413 | 484 |
| LPS (10 µg/ml) | Ibuprofen (50 µM) | 427 | 466 |
| LPS (10 µg/ml) | Naproxen (50 µM) | 409 | 458 |

| | | | |
|---|---|---|---|
| ^{#}Data are expressed as mean of duplicates | | | |

Analgesics inhibit PGE2 responses of normal human bladder smooth muscle cells to inflammatory and non- inflammatory stimuli - Consistent with the induction of COX2 responses described above, both carbachol and LPS induced production of PGE2 by normal human bladder smooth muscle cells. Acetaminophen, aspirin, ibuprofen and naproxen were also found to suppress the LPS-induced PGE2 responses at either 5 µM or 50 µM (Table 7).

**Table 7. PGE2 secretion by normal human bladder smooth muscle cells after in vitro stimulation with inflammatory and non- inflammatory stimuli and treatment with analgesic**

| **Stimuli** | **Analgesic** | **PGE2 levels^{#} (pg/ml)** | **PGE2 levels (pg/ml)** |
|---|---|---|---|
| | | **Subject 1** | **Subject 2** |
| None | None | < 20.5 | < 20.5 |
| Carbachol 10⁻³ M | Acetaminophen (50 µM) | 129 | 104 |
| Carbachol 10⁻³ M | Aspirin (50 µM) | 76 | 62 |
| Carbachol 10⁻³ M | Ibuprofen (50 µM) | 89 | 59 |
| Carbachol 10⁻³ M | Naproxen (50 µM) | 84 | 73 |
| Carbachol 10⁻³ M | Acetaminophen (50 µM) | 77 | 66 |
| | | | |
| LPS (10 µg/ml) | None | 1125 | 998 |
| LPS (10 µg/ml) | Acetaminophen (5 µM) | 817 | 542 |
| LPS (10 µg/ml) | Aspirin (5 µM) | 838 | 598 |
| LPS (10 µg/ml) | Ibuprofen (5 µM) | 824 | 527 |
| LPS (10 µg/ml) | Naproxen (5 µM | 859 | 506 |
| LPS (10 µg/ml) | Acetaminophen (50 µM) | 803 | 540 |
| LPS (10 µg/ml) | Aspirin (50 µM) | 812 | 534 |
| LPS (10 µg/ml) | Ibuprofen (50 µM) | 821 | 501 |
| LPS (10 µg/ml) | Naproxen (50 µM) | 819 | 523 |

| | | | |
|---|---|---|---|
| ^{#}Data are expressed as mean of duplicates | | | |

*Analgesics inhibit cytokine responses of normal human bladder cells to an inflammatory stimuli* - Analysis of cells and culture supernatants after 24 hours of cultures showed that none the analgesics tested alone induced IL-6 or TNFα secretion in normal human bladder smooth muscle cells. As shown in Tables 8 and 9, the dose of carbachol tested induced low, but significant TNFα and IL-6 responses in normal human bladder smooth muscle cells. On the other hand, LPS treatment resulted in massive induction of these proinflammatory cytokines. Acetaminophen, aspirin, ibuprofen and naproxen suppress the effect of carbachol and LPS on TNFα and IL-6 responses. The suppressive effect of the analgesic on LPS-induced responses was seen when these drugs were tested at either 5 µM or 50 µM.

**Table 8. TNFα secretion by normal human bladder smooth muscle cells after in vitro stimulation with inflammatory and non- inflammatory stimuli and treatment with analgesic**

| **Stimuli** | **Analgesic** | **TNFα (pg/ml)^{#}** | **TNFα (pg/ml)** |
|---|---|---|---|
| | | **Subject 1** | **Subject 2** |
| None | None | < 5 | <5 |
| Carbachol 10⁻³ M | None | 350 | 286 |
| Carbachol 10⁻³ M | Acetaminophen (50 µM) | 138 | 164 |
| Carbachol 10⁻³ M | Aspirin (50 µM) | 110 | 142 |
| Carbachol 10⁻³ M | Ibuprofen (50 µM) | 146 | 121 |
| Carbachol 10⁻³ M | Naproxen (50 µM) | 129 | 137 |
| | | | |
| LPS (10 µg/ml) | None | 5725 | 4107 |
| LPS (10 µg/ml) | Acetaminophen (5 µM) | 2338 | 2267 |
| LPS (10 µg/ml) | Aspirin (5 µM) | 2479 | 2187 |
| LPS (10 µg/ml) | Ibuprofen (5 µM) | 2733 | 2288 |
| LPS (10 µg/ml) | Naproxen (5 µM | 2591 | 2215 |
| LPS (10 µg/ml) | Acetaminophen (50 µM) | 2184 | 2056 |
| LPS (10 µg/ml) | Aspirin (50 µM) | 2266 | 2089 |
| LPS (10 µg/ml) | Ibuprofen (50 µM) | 2603 | 1997 |
| LPS (10 µg/ml) | Naproxen (50 µM) | 2427 | 2192 |

| | | | |
|---|---|---|---|
| ^{#}Data are expressed as mean of duplicates. | | | |

**Table 9. IL-6 secretion by normal human bladder smooth muscle cells after in vitro stimulation with inflammatory and non- inflammatory stimuli and treatment with analgesic**

| **Stimuli** | **Analgesic** | **IL-6 (pg/ml)^{#}** | **IL-6 (pg/ml)** |
|---|---|---|---|
| | | **Subject 1** | **Subject 2** |
| None | None | < 5 | <5 |
| Carbachol 10⁻³M | None | 232 | 278 |
| Carbachol 10⁻³ M | Acetaminophen (50 µM) | 119 | 135 |
| Carbachol 10⁻³ M | Aspirin (50 µM) | 95 | 146 |
| Carbachol 10⁻³ M | Ibuprofen (50 µM) | 107 | 118 |
| Carbachol 10⁻³ M | Naproxen (50 µM) | 114 | 127 |
| | | | |
| LPS (10 µg/ml) | None | 4838 | 4383 |
| LPS (10 µg/ml) | Acetaminophen (5 µM) | 2012 | 2308 |
| LPS (10 µg/ml) | Aspirin (5 µM) | 2199 | 2089 |
| LPS (10 µg/ml) | Ibuprofen (5 µM) | 2063 | 2173 |
| LPS (10 µg/ml) | Naproxen (5 µM | 2077 | 2229 |
| LPS (10 µg/ml) | Acetaminophen (50 µM) | 2018 | 1983 |
| LPS (10 µg/ml) | Aspirin (50 µM) | 1987 | 2010 |
| LPS (10 µg/ml) | Ibuprofen (50 µM) | 2021 | 1991 |
| LPS (10 µg/ml) | Naproxen (50 µM) | 2102 | 2028 |

| | | | |
|---|---|---|---|
| ^{#}Data are expressed as mean of duplicates | | | |

Primary normal human bladder smooth muscle cells were isolated, cultured and evaluated for their responses to analgesics in the presence of non-inflammatory (carbachol) and inflammatory (LPS) stimuli. The goal of this study was to determine whether or not normal human bladder smooth muscle cells recapitulate the observations previously made with murine bladder cells.

The above-described experiment will be repeated with analgesic agents and/or antimuscarinic agents in delayed-release, or extended-release formulation or delayed-and-extended-release formulations.

The above description is for the purpose of teaching the person of ordinary skill in the art how to practice the present invention, and it is not intended to detail all those obvious modifications and variations of it which will become apparent to the skilled worker upon reading the description. It is intended, however, that all such obvious modifications and variations be included within the scope of the present invention, which is defined by the following claims. The claims are intended to cover the claimed components and steps in any sequence which is effective to meet the objectives there intended, unless the context specifically indicates the contrary.

## Claims

1. A pharmaceutical composition for use in therapeutically reducing the frequency of urination, comprising a first analgesic agent,
wherein said first analgesic agent is acetaminophen, wherein said pharmaceutical composition is formulated in a delayed-release formulation having an enteric coating, and wherein said first analgesic agent is administered orally at a daily dose of 5 mg to 2000 mg.

2. The pharmaceutical composition of Claim 1, for use in therapeutically reducing the frequency of urination, wherein said delayed-release formulation comprises a material selected from the group consisting of hydroxypropyl methylcellulose, methylcellulose, methyl hydroxyethylcellulose, hydroxypropylcellulose, carboxymethylcellulose, acrylate copolymers, polyethylene glycols, polyvinylpyrrolidone, methacrylic acid copolymer, cellulose acetate phthalate, hydroxypropyl methylcellulose phthalate, hydroxypropyl methylcellulose acetate succinate, polyvinyl acetate phthalate, shellac and ethylcellulose.

3. The pharmaceutical composition of Claim 2, for use in therapeutically reducing the frequency of urination, wherein said delayed-release formulation further comprises glyceryl monostearate or glyceryl distearate.

4. The pharmaceutical composition of Claim 1, for use in therapeutically reducing the frequency of urination, wherein said delayed-release formulation comprises a water insoluble capsule body closed at one end with an insoluble, but permeable and swellable hydrogel plug, wherein said plug comprises a material selected from the group consisting of polymethacrylates, erodible compressed polymers, congealed melted polymer and enzymatically controlled erodible polymers.

5. The pharmaceutical composition of Claim 1, for use in therapeutically reducing the frequency of urination, wherein said first analgesic agent is administered orally at a daily dose of;
a) 50 mg to 500 mg,
b) 100 mg to 500 mg, or
c) 250 mg to 500 mg.

6. The pharmaceutical composition of Claim 1, for use in therapeutically reducing the frequency of urination, wherein said first analgesic agent is administered orally at a daily dose of 250 mg to 1000 mg.

7. The pharmaceutical composition of Claim 1, for use in therapeutically reducing the frequency of urination, wherein said pharmaceutical composition further comprises:
a second analgesic agent selected from the group consisting of aspirin, ibuprofen and naproxen,
wherein said second analgesic agent is administered orally at a daily dose of;
a) 5 mg to 2000 mg,
b) 50 mg to 500 mg,
c) 100 mg to 500 mg, or
d) 250 mg to 500 mg.

8. The pharmaceutical composition of Claim 8, for use in therapeutically reducing the frequency of urination, wherein said second analgesic agent is administered orally at a daily dose of 250 mg to 1000 mg.

9. The pharmaceutical composition of Claim 1, for use in therapeutically reducing the frequency of urination, wherein said pharmaceutical composition further comprises one or more antimuscarinic agents selected from the group consisting of oxybutynin, solifenacin, darifenacin and atropine, one or more antidiuretics, or one or more spasmolytics.

10. The pharmaceutical composition of Claim 1, for use in therapeutically reducing the frequency of urination, to be administered with a diuretic,
wherein said diuretic is administered at least 8 hours prior to a target time, and wherein said pharmaceutical composition is administered within 2 hours prior to said target time.

11. The pharmaceutical composition of Claim 10, for use in therapeutically reducing the frequency of urination wherein said pharmaceutical composition is used for treating a subject with nocturia.

## Patentansprüche

1. Pharmazeutische Zusammensetzung zur Verwendung in der therapeutischen Verringerung der Miktionsfrequenz, umfassend ein erstes Analgetikum, wobei das erste Analgetikum Acetaminophen ist, wobei die pharmazeutische Zusammensetzung in einer Formulierung zur verzögerten Freisetzung mit einer magensaftresistenen Beschichtung vorliegt, und wobei das erste Analgetikum oral in einer Tagesdosis von 5 mg bis 2000 mg verabreicht wird.

2. Pharmazeutische Zusammensetzung nach Anspruch 1 zur Verwendung in der therapeutischen Verringerung der Miktionsfrequenz, wobei die Formulierung zur verzögerten Freisetzung ein Material ausgewählt aus der Gruppe bestehend aus HydroxypropylMethylcellulose, Methylcellulose, Methylhydroxyethylcellulose, Hydroxypropylcellulose, Carboxymethylcellulose, Acrylat-Copolymere, Polyethylenglykole, Polyvinylpyrrolidon, Methacrylsäure-Copolymer, Celluloseacetatphthalat, Hydroxypropyl-Methylcellulosephthalat, Hydroxypropyl-Methylcellulose-Acetat-Succinat, Polyvinylacetatphthalat, Schellack und Ethylcellulose umfasst.

3. Pharmazeutische Zusammensetzung nach Anspruch 2 zur Verwendung in der therapeutischen Verringerung der Miktionsfrequenz, wobei die Formulierung zur verzögerten Freisetzung ferner Glycerylmonostearat oder Glyceryldistearat umfasst.

4. Pharmazeutische Zusammensetzung nach Anspruch 1 zur Verwendung in der therapeutischen Verringerung der Miktionsfrequenz, wobei die Formulierung zur verzögerten Freisetzung einen nicht wasserlöslichen Kapselkörper umfasst, der an einem Ende mit einem unlöslichen, aber durchlässigen und quellfähigen Hydrogelstopfen verschlossen ist, wobei der Stopfen ein Material aus der Gruppe bestehend aus Polymethacrylaten, erodierbaren komprimierten Polymeren, erstarrtem geschmolzenem Polymer und enzymatisch gesteuert erodierbaren Polymeren ausgewählt ist.

5. Pharmazeutische Zusammensetzung nach Anspruch 1 zur Verwendung in der therapeutischen Verringerung der Miktionsfrequenz, wobei das erste Analgetikum oral in einer Tagesdosis von:
a) 50 mg bis 500 mg,
b) 100 mg bis 500 mg, oder
c) 250 mg bis 500 mg verabreicht wird.

6. Pharmazeutische Zusammensetzung nach Anspruch 1 zur Verwendung in der therapeutischen Verringerung der Miktionsfrequenz, wobei das erste Analgetikum oral in einer Tagesdosis von 250 mg bis 1000 mg verabreicht wird.

7. Pharmazeutische Zusammensetzung nach Anspruch 1 zur Verwendung in der therapeutischen Verringerung der Miktionsfrequenz, wobei die pharmazeutische Zusammensetzung ferner umfasst:
ein zweites Analgetikum ausgewählt aus der Gruppe bestehend aus Aspirin, Ibuprofen und Naproxen,
wobei das zweite Analgetikum oral in einer Tagesdosis von:
a) 5 mg bis 2000 mg,
b) 50 mg bis 500 mg,
c) 100 mg bis 500 mg, oder
d) 250 mg bis 500 mg verabreicht wird.

8. Pharmazeutische Zusammensetzung nach Anspruch 8 zur Verwendung in der therapeutischen Verringerung der Miktionsfrequenz, wobei das zweite Analgetikum oral in einer Tagesdosis von 250 mg bis 1000 mg verabreicht wird.

9. Pharmazeutische Zusammensetzung nach Anspruch 1 zur Verwendung in der therapeutischen Verringerung der Miktionsfrequenz, wobei die pharmazeutische Zusammensetzung ferner ein oder mehrere Antimuskarinika ausgewählt aus der Gruppe bestehend aus Oxybutynin, Solifenacin, Darifenacin und Atropin, ein oder mehrere Antidiuretika, oder ein oder mehrere Spasmolytika umfasst.

10. Pharmazeutische Zusammensetzung nach Anspruch 1 zur Verwendung in der therapeutischen Verringerung der Miktionsfrequenz, wobei die Zusammensetzung zusammen mit einem Diuretikum verabreicht werden soll, wobei das Diuretikum mindestens 8 Stunden vor einer Zielzeit verabreicht wird, und wobei die pharmazeutische Zusammensetzung innerhalb von 2 Stunden vor der der Zielzeit verabreicht wird.

11. Pharmazeutische Zusammensetzung nach Anspruch 10 zur Verwendung in der therapeutischen Verringerung der Miktionsfrequenz, wobei die pharmazeutische Zusammensetzung zur Behandlung eines Subjekts mit Nokturie verwendet wird.

## Revendications

1. Composition pharmaceutique pour une utilisation dans la réduction thérapeutique de la fréquence des mictions, comprenant un premier agent analgésique,
dans laquelle ledit premier agent analgésique est l'acétaminophène, dans laquelle ladite composition pharmaceutique est formulée dans une formulation à libération retardée ayant un enrobage entérique, et dans laquelle ledit premier agent analgésique est administré par voie orale à une dose journalière allant de 5 mg à 2000 mg.

2. Composition pharmaceutique de la revendication 1, pour une utilisation dans la réduction thérapeutique de la fréquence des mictions, dans laquelle ladite formulation à libération retardée comprend un matériau choisi dans le groupe constitué par l'hydroxypropylméthylcellulose, la méthylcellulose, l'hydroxyéthylcellulose de méthyle, l'hydroxypropylcellulose, la carboxyméthylcellulose, des copolymères d'acrylate, des polyéthylène glycols, le polyvinylpyrrolidone, un copolymère d'acide méthacrylique, le phtalate d'acétate de cellulose, le phtalate d'hydroxypropylméthylcellulose, le succinate d'acétate d'hydroxypropylméthylcellulose, le phtalate d'acétate de polyvinyle, la gomme-laque et l'éthylcellulose.

3. Composition pharmaceutique de la revendication 2, pour une utilisation dans la réduction thérapeutique de la fréquence des mictions, dans laquelle ladite formulation à libération retardée comprend en outre du monostéarate de glycéryle ou du distéarate de glycéryle.

4. Composition pharmaceutique de la revendication 1, pour une utilisation dans la réduction thérapeutique de la fréquence des mictions, dans laquelle ladite formulation à libération retardée comprend un corps de capsule insoluble dans l'eau fermé à une extrémité avec un bouchon d'hydrogel insoluble, mais perméable et gonflable, dans laquelle ledit bouchon comprend un matériau choisi dans le groupe constitué par des polyméthacrylates, des polymères comprimés érodables, un polymère figé fondu et des polymères érodables régulés de manière enzymatique.

5. Composition pharmaceutique de la revendication 1, pour une utilisation dans la réduction thérapeutique de la fréquence des mictions, dans laquelle ledit premier agent analgésique est administré par voie orale à une dose journalière allant ;
a) de 50 mg à 500 mg,
b) de 100 mg à 500 mg, ou
c) de 250 mg à 500 mg.

6. Composition pharmaceutique de la revendication 1, pour une utilisation dans la réduction thérapeutique de la fréquence des mictions, dans laquelle ledit premier agent analgésique est administré par voie orale à une dose journalière allant de 250 mg à 1000 mg.

7. Composition pharmaceutique de la revendication 1, pour une utilisation dans la réduction thérapeutique de la fréquence des mictions, dans laquelle ladite composition pharmaceutique comprend en outre :
un deuxième agent analgésique choisi dans le groupe constitué par l'aspirine, l'ibuprofène et le naproxène,
dans laquelle ledit deuxième agent analgésie est administré par voie orale à une dose journalière allant ;
a) de 5 mg à 2000 mg,
b) de 50 mg à 500 mg,
c) de 100 mg à 500 mg, ou
d) de 250 mg à 500 mg.

8. Composition pharmaceutique de la revendication 8, pour une utilisation dans la réduction thérapeutique de la fréquence des mictions, dans laquelle ledit deuxième agent analgésique est administré par voie orale à une dose journalière allant de 250 mg à 1000 mg.

9. Composition pharmaceutique de la revendication 1, pour une utilisation dans la réduction thérapeutique de la fréquence des mictions, dans laquelle ladite composition pharmaceutique comprend en outre un ou plusieurs agent(s) anti-muscarinique(s) choisi(s) dans le groupe constitué par l'oxybutynine, la solifénacine, la darifénacine et l'atropine, un ou plusieurs agent(s) antidiurétique(s), ou un ou plusieurs spasmolytique(s).

10. Composition pharmaceutique de la revendication 1, pour une utilisation dans la réduction thérapeutique de la fréquence des mictions, à administrer avec un diurétique,
dans laquelle ledit diurétique est administré au moins 8 heures avant un moment cible et dans laquelle ladite composition pharmaceutique est administrée dans les 2 heures avant ledit moment cible.

11. Composition pharmaceutique de la revendication 10, pour une utilisation dans la réduction thérapeutique de la fréquence des mictions, dans laquelle ladite composition pharmaceutique est utilisée pour traiter un sujet atteint de nycturie.
